# EUROPÄISCHE PATENTANMELDUNG

(11) **EP 2 606 967 A1**
(43) Veröffentlichungstag der Anmeldung: **26.06.2013**
(21) Anmeldenummer: 11194327.0
(22) Anmeldetag: 19.12.2011
(51) Int. Cl.: B01J 23/30, B01J 35/00, B01J 35/02, B01J 37/00, B01J 35/10, C07C 319/08, C07C 321/04

(54) **Katalysator zur Synthese von Alkylmercaptanen und Verfahren zu seiner Herstellung**

(71) Anmelder: Evonik Degussa GmbH, 45128 Essen (DE)
(72) Erfinder: Fonfe, Benjamin, Dr., 60318 Frankfurt (DE); Fuß, Sebastian, 36103 Flieden (DE); Wilz, Frank, 63755 Alzenau (DE); Jakob, Harald, Dr., 63594 Hasselroth (DE); Weckbecker, Christoph, Dr,, 63584 Gründau-Lieblos (DE)

(57) **Zusammenfassung**

Die vorliegende Erfindung betrifft einen Katalysator, welcher ein Trägermaterial und eine oxidische Zusammensetzung, enthaltend mindestens ein Alkalimetall und Wolfram umfasst, ein Verfahren zur Herstellung derartiger Katalysatoren sowie ein Verfahren zur Herstellung von Alkylmercaptanen durch Umsetzung von Alkanolen mit Schwefelwasserstoff in Gegenwart eines solchen Katalysators.

## Beschreibung

Die vorliegende Erfindung betrifft einen Katalysator, welcher ein Trägermaterial und eine oxidische Zusammensetzung, enthaltend mindestens ein Alkalimetall und Wolfram umfasst, ein Verfahren zur Herstellung derartiger Katalysatoren sowie ein Verfahren zur Herstellung von Alkylmercaptanen durch Umsetzung von Alkanolen mit Schwefelwasserstoff in Gegenwart eines solchen Katalysators.

Insbesondere Methylmercaptan ist ein industriell wichtiges Zwischenprodukt, beispielsweise für die Synthese von Methionin, Dimethylsulfoxid oder Dimethylsulfon. Methylmercaptan wird heutzutage aus Methanol und Schwefelwasserstoff durch Umsetzung in Gegenwart eines Katalysators aus Aluminiumoxid hergestellt. Die Synthese des Methylmercaptans erfolgt gewöhnlich in der Gasphase bei Temperaturen zwischen 300 und 500 °C und einem Druck im Bereich zwischen 1 und 25 bar.

Das hierbei erhaltene Reaktionsgemisch enthält neben dem gewünschten Produkt Methylmercaptan auch nicht umgesetzte Edukte und Nebenprodukte, beispielsweise Dimethylsulfid und Dimethylether, sowie im Sinne der Reaktion inerte Gase, beispielsweise Methan, Kohlenmonoxid, Wasserstoff und Stickstoff. Aus diesem Reaktionsgemisch muss das gebildete Methylmercaptan abgetrennt werden.

Für die Wirtschaftlichkeit des Verfahrens sind daher sowohl ein hoher Umsatz als auch eine hohe Selektivität der katalytischen Reaktion von Methanol und Schwefelwasserstoff zu Methylmercaptan erforderlich, um den Aufwand bei der Abtrennung des gebildeten Methylmercaptans aus dem Reaktionsgemisch so gering wie möglich zu halten. Die Abtrennung aus dem Reaktionsgasgemisch erfolgt üblicherweise durch Kondensation des Methylmercaptans, wobei insbesondere der Energieaufwand für die Kühlung des Reaktionsgemisches einen großen Kostenfaktor darstellt.

Zur Erhöhung der Aktivität und Selektivität des Katalysators wird Aluminiumoxid üblicherweise mit einem Alkalimetallwolframat versetzt (teilweise auch als Promoter bezeichnet), beispielsweise mit Kalium- oder Cäsiumwolframat. Der Anteil des Wolframats, bezogen auf das Gesamtgewicht des Katalysators, beträgt hierbei üblicherweise bis zu etwa 20 %, wie dies beispielsweise in dem Patent US 2,820,062 beschrieben ist.

Unter Zuhilfenahme spezieller Verfahren zur Herstellung des Katalysators, bei welchen das Trägermaterial mehrfach mit einer Lösung des Alkalimetallwolframats in einem vergleichsweise aufwendigen Prozess imprägniert wird, kann der Anteil des Alkalimetallwolframats in dem Katalysator auf 25 Gew.-% und mehr gesteigert werden (siehe beispielsweise EP 0 832 878 A2 und DE 103 38 887 A1), wobei die Erhöhung der Konzentration des Alkalimetallwolframats oberhalb eines Anteils von ungefähr 25 Gew.-% lediglich eine Steigerung der Selektivität bei gleichzeitig geringerer Aktivität bewirkt, sofern das Alkalimetall und Wolfram in der oxidischen Zusammensetzung in einem stöchiometrischen Verhältnis von 2:1 auf den Katalysator aufgebracht werden (siehe DE 103 38 887 A1). Durch Verwendung eines nicht stöchiometrischen Verhältnisses von Cäsium und Wolfram in der zur Imprägnierung verwendeten Lösung kann die Beladung zwar auf bis zu über 35 Gew.-% erhöht werden, bei solch hohen Beladungen kann jedoch keine signifikante Steigerung des Umsatzes bzw. der Selektivität mehr verzeichnet werden; insbesondere bei Beladungen oberhalb von 45 Gew.-%, bezogen auf das Gesamtgewicht des Katalysators sinken Umsatz bzw. Selektivität sogar.

Aufgabe der vorliegenden Erfindung war es daher, einen Katalysator sowie ein Verfahren zu seiner Herstellung bereitzustellen, welcher gegenüber den aus dem Stand der Technik bekannten Katalysatoren zur Umsetzung von Schwefelwasserstoff mit Alkanolen, insbesondere mit Methanol, zu Alkylmercaptanen, insbesondere Methylmercaptan, eine verbesserte Aktivität und Selektivität gegenüber den bekannten Katalysatoren aufweist. Die verbesserte Aktivität und Selektivität sollte bei allen üblicherweise in Syntheseverfahren zur Herstellung von Alkylmercaptanen eingesetzten Molverhältnissen von Schwefelwasserstoff zu Methanol vorliegen, beispielsweise im Bereich von H₂S: MeOH ≥1:1 bis ≤ 10:1 und insbesondere auch bei vergleichsweise geringen Molverhältnissen von Schwefelwasserstoff zu Methanol, d. h. bei einem Verhältnis von H₂S:MeOH ≤ 3:1, bevorzugt ≤ 2:1.

Überraschenderweise ist nun gefunden worden, dass diese Aufgabe gelöst wird durch ein Verfahren zur Herstellung eines Katalysators, umfassend ein Trägermaterial und eine oxidische Zusammensetzung, welche neben Sauerstoff mindestens ein Alkalimetall und Wolfram enthält, besagtes Verfahren umfassend die Schritte:
1) Bereitstellen mindestens eines Trägermaterials, einer oxidischen Wolframverbindung und mindestens einer separaten Alkalimetallverbindung,
2) Vermischen des Trägermaterials mit der oxidischen Wolframverbindung und der mindestens einen separaten Alkalimetallverbindung, um eine Katalysatormasse zu erhalten und
3) Formen der erhaltenen Katalysatormasse
sowie die nach diesem Verfahren hergestellten Katalysatoren.

In den aus dem Stand der Technik bekannten Verfahren, welche ein Alkalimetallwolframat und ein Trägermaterial wie beispielsweise Aluminiumoxid enthalten, wird stets zunächst eine Lösung des Alkalimetallwolframats hergestellt, welche dann zu dem Trägermaterial gegeben wird. Diese Lösung wird dann üblicherweise in einem Imprägnierverfahren, vorzugsweise einem mehrstufigen Imprägnierverfahren, auf Teilchen des Trägermaterials aufgebracht, die eine Größe von mehreren Millimetern haben.

Überraschenderweise ist nun gefunden worden, dass eine gesteigerte Aktivität und Selektivität des Katalysators erhalten werden kann, wenn das Trägermaterial nicht mit einer Lösung, welche bereits sowohl das Alkalimetall als auch das Wolframat enthält, imprägniert wird, sondern das Trägermaterial mit einer oxidischen Wolframverbindung, die vorzugsweise kein Alkalimetall enthält und einer separaten Alkalimetallverbindung vermischt wird.

Mit den nach dem erfindungsgemäßen Verfahren hergestellten Katalysatoren lassen sich bei identischen Reaktionsbedingungen und gleicher Beladung im Vergleich zu den aus dem Stand der Technik bekannten Katalysatoren, welche nach dem Imprägnierverfahren hergestellt sind, ein höherer Umsatz und eine größere Selektivität erzielen.

Das erfindungsgemäße Verfahren ermöglicht weiterhin eine einfache Variation des Verhältnisses des Gehaltes an Alkalimetall zu dem Gehalt an Wolfram in der oxidischen Zusammensetzung, ausgedrückt als Verhältnis von Alkalimetalloxid A₂O in Gew.-%, bezogen auf die Gesamtmasse des Katalysators, zu Wolfram(VI)oxid WO₃ in Gew.-%, bezogen auf die Gesamtmasse des Katalysators. Die Gesamtbeladung des Katalysators mit der oxidischen Zusammensetzung, welche das Alkalimetall und Wolfram enthält, ist die Summe der zuvor genannten Anteile von A₂O und WO₃ in dem Katalysator. Die Vorteile eines nicht stöchiometrischen Verhältnisses sind bereits in der Anmeldung DE 103 38 887 A1 beschrieben.

Nach dem erfindungsgemäßen Verfahren ist es ferner möglich, hohe Beladungen des Trägermaterials mit der oxidischen Zusammensetzung, welche das Alkalimetall und Wolfram enthält, zu erhalten, ohne dass ein mehrstufiges, sehr zeitaufwendiges Imprägnierverfahren durchgeführt werden müsste.

Weiterhin ist es mit dem vorliegenden Verfahren sogar möglich, Katalysatoren zu erhalten, welche eine Beladung von über 45 Gew.-% der oxidischen Zusammensetzung enthalten, die sowohl eine gute Aktivität als auch eine gute Selektivität zeigen.

Ein weiterer Vorteil der Katalysatoren der vorliegenden Erfindung ist die Tatsache, dass mit ihnen Katalysatoren zur Verfügung stehen, mit welchen in der Reaktion von Alkanolen und Schwefelwasserstoff zu Alkylmercaptanen unter typischen Reaktionsbedingungen Selektivitäten von über 95 % bei einem Umsatz von ebenfalls über 95 % erzielt werden können. Mit den erfindungsgemäßen Katalysatoren lässt sich beispielsweise bei typischen Reaktionsbedingungen (Temperatur etwa 300 bis 370 °C, Druck etwa 9 bar, Massenverhältnis H₂S/MeOH etwa 1.9) die Reaktion von Schwefelwasserstoff und Methanol zu Methylmercaptan mit einer Selektivität von deutlich über 95 % bei einem Umsatz von ebenfalls über 95 % durchführen, wie die erfindungsgemäßen Beispiele belegen. Mit den erfindungsgemäßen Katalysatoren lässt sich selbst bei einem Verhältnis von H₂S : MeOH < 2:1 teilweise sogar bei einem Umsatz von über 99 °C % eine Selektivität von über 96 % erzielen. Bei höheren Verhältnissen von H₂S : MeOH, d. h. H₂S : MeOH > 2:1, sind die Ergebnisse noch besser.

Die hohe Selektivität bei hohem Umsatz und vergleichsweise geringen Verhältnissen von H₂S : MeOH (beispielsweise ≤ 3:1) ermöglicht unter anderem eine effiziente Synthese von Alkylmercaptanen bei einem Umsatz ≥ 98 %, wodurch auch die Trennung des Alkylmercaptans aus dem Reaktionsgemisch erheblich vereinfacht wird.

Im Sinne der vorliegenden Erfindung bedeutet der Begriff Umsatz den Quotienten aus der im Laufe der katalysierten Reaktion umgesetzten, d.h. tatsächlich reagiert habenden Menge an Methanol zu der eingesetzten Menge an Methanol. Dieser kann beispielsweise gaschromatografisch bestimmt werden.

Im Sinne der vorliegenden Erfindung bedeutet der Begriff Selektivität den Quotienten aus der gebildeten Menge Methylmercaptan zu der umgesetzten Menge Methanol.

Die oxidische Zusammensetzung, welche neben Sauerstoff mindestens ein Alkalimetall und Wolfram enthält, umfasst im Sinne der vorliegenden Erfindung sowohl Alkalimetallwolframate mit stöchiometrischen Verhältnissen von Alkalimetall und Wolfram, wie beispielsweise A₂WO₄ (bzw. A₂O + WO₃), in der A ein Alkalimetall bedeutet, aber auch Mischoxide enthaltend Wolfram und mindestens ein Alkalimetall der allgemeinen Formel AₓWO_{y}, in der bedeuten A: mindestens ein Alkalimetall, bevorzugt ausgewählt aus der Gruppe, bestehend aus Natrium, Kalium, Rubidium und Cäsium,
x: molarer Anteil des mindestens einen Alkalimetalls im Verhältnis zu Wolfram in der Zusammensetzung, welcher bevorzugt im Bereich von 2:1 zu 0.8:1 liegt, besonders bevorzugt im Bereich von 1,9:1 zu 1,1:1, und
y: molarer Anteil Sauerstoff in der Zusammensetzung, welcher bevorzugt im Bereich von 3,4 bis 4 liegt,
sowie innige Vermischungen von Alkalimetalloxiden und oxidischen Wolframverbindungen. Als Mischoxide werden im Sinne der vorliegenden Erfindung Substanzen bezeichnet, welche Oxide mehrerer unterschiedlicher chemischer Elemente in inniger Vermischung auf molekularer Ebene enthalten.

Eine separate Alkalimetallverbindung bedeutet im Sinne der vorliegenden Erfindung eine chemische Verbindung, enthaltend mindestens ein Alkalimetall, die räumlich getrennt von der oxidischen Wolframverbindung vorliegt, d. h. insbesondere nicht in Form einer Lösung, welche neben Alkalimetallionen eine Wolframverbindung bzw. Wolframionen enthält.

Die in dem erfindungsgemäßen Verfahren eingesetzte oxidische Wolframverbindung ist bevorzugt eine Verbindung, die Wolfram in der Oxidationsstufe VI enthält. Die oxidische Wolframverbindung ist bevorzugt ausgewählt aus der Gruppe, bestehend aus Wolframtrioxid (WO₃), Wolframsäure (WO₃ • H₂O), Metawolframsäure, Parawolframsäure, Isopolywolframsäuren, Ammoniumsalzen dieser Wolframsäuren, d. h. Ammoniumwolframaten (Ortho-, Meta- und Parawolframate) und Ammoniumisopolywolframaten, Heteropolywolframaten, Hydraten derselben oder Mischungen derselben und stellt bevorzugt Wolframsäure oder Ammoniumortho-, -meta- oder ―parawolframat dar.

Die mindestens eine in dem erfindungsgemäßen Verfahren eingesetzte Alkalimetallverbindung ist bevorzugt eine wasserlösliche Alkalimetallverbindung. Die Alkalimetallverbindung ist weiterhin bevorzugt eine basische Alkalimetallverbindung.

Als basische Alkalimetallverbindung im Sinne der vorliegenden Erfindung werden wasserlösliche Verbindungen bezeichnet, welche ein Alkalimetall enthalten und beim Lösen in Wasser eine Lösung mit einem pH-Wert von pH > 7 bei Raumtemperatur (23 °C) ergeben. Bevorzugt ist die Alkalimetallverbindung ausgewählt aus der Gruppe bestehend aus Hydroxiden und Carbonaten von Alkalimetallen. Bevorzugt stellt das Alkalimetall mindestens ein Alkalimetall ausgewählt aus der Gruppe bestehend aus Natrium, Kalium, Cäsium und Rubidium oder eine Mischung mindestens zweier dieser Alkalimetalle dar. Besonders bevorzugt ist die basische Alkalimetallverbindung ein Hydroxid oder Carbonat eines Alkalimetalls, ausgewählt aus der Gruppe bestehend aus Natrium, Kalium, Cäsium und Rubidium. Insbesondere bevorzugt ist im Sinne der vorliegenden Erfindung die Verwendung von Kaliumhydroxid, Cäsiumhydroxid oder einer Mischung von Kaliumhydroxid und Cäsiumhydroxid.

Das Trägermaterial in dem erfindungsgemäßen Katalysator ist bevorzugt ein oxidisches anorganisches Trägermaterial. Bevorzugt ist dieses ausgewählt aus der Gruppe enthaltend Aluminiumoxid, Siliciumdioxid, Titandioxid, Zirconoxid, amorphe Aluminosilikate und Gemische derselben. Bevorzugt wird dieses Trägermaterial in Form von Partikeln mit einer Größe von weniger als 1000 µm eingesetzt. Besonders bevorzugt stellt das Trägermaterial Aluminiumoxid dar.

Die geometrische Form der Trägermaterialpartikel ist hierbei grundsätzlich nicht beschränkt. Die Partikel können beispielsweise in Form von Kugeln, Zylindern, Quadern, Würfeln, Prismen, Ellipsoiden, Ringen oder auch unregelmäßig geformten Teilchen vorliegen, ohne auf diese Formen beschränkt zu sein. Als Größe dieser Partikel wird im Sinne der vorliegenden Erfindung die jeweils größte Längenausdehnung eines Partikels verstanden, d. h. beispielsweise bei quaderförmigen Partikeln die Länge und bei kugelförmigen Partikeln der Durchmesser. Die Größe der in dem erfindungsgemäßen Verfahren verwendeten Partikel beträgt weiterhin bevorzugt weniger als 500 µm, besonders bevorzugt weniger als 250 µm, besonders bevorzugt weniger als 100 µm und insbesondere weniger als 50 µm. Ganz besonders bevorzugt liegt die Größe der verwendeten Partikel des Trägermaterials im Bereich von 1 bis 25 µm.

Besonders bevorzugt als Trägermaterial ist sogenanntes aktives Aluminiumoxid, welches eine hohe spezifische Oberfläche im Bereich von etwa 10 bis etwa 400 m²/g aufweist und hauptsächlich aus Oxiden der Übergangsreihe der kristallographischen Phasen des Aluminiumoxids besteht (siehe Ullmann's Encyclopaedia of Industrial Chemistry, 1985, Vol. A1, 561-562). Zu diesen Übergangsoxiden gehören γ-, δ-, ε-, κ-, χ- und θ-Aluminiumoxid. Aktives Aluminiumoxid wird kommerziell für katalytische Anwendungen in verschiedenen Qualitäten und Lieferformen angeboten. Besonders geeignet für die Herstellung der erfindungsgemäßen Katalysatoren sind Pulver des entsprechenden Aluminiumoxids aus Partikeln mit einer Teilchengröße von etwa 1 bis 25 µm, einer spezifischen Oberfläche im Bereich von 180 bis 400 m²/g, bestimmt gemäß ISO 9277, einem Mesoporenvolumen (d = 2-50 nm) im Bereich von 5 bis 50 ml/100g, bestimmt gemäß DIN 66134, einem Makroporenvolumen (d > 50nm) im Bereich von 20 bis 100 ml/100 g , bestimmt gemäß DIN 66133 und einer Schüttdichte im Bereich von 300 bis 1000 g/l, bestimmt gemäß DIN ISO 697.

Bevorzugt werden in dem erfindungsgemäßen Verfahren die oxidische Wolframverbindung und die separate Alkalimetallverbindung nacheinander zu dem Trägermaterial hinzugegeben. Hierbei kann das Trägermaterial sowohl zuerst mit der oxidischen Wolframverbindung vermischt werden und das erhaltene Gemisch danach mit der mindestens einen Alkalimetallverbindung vermischt werden. Alternativ kann das Trägermaterial zuerst mit der mindestens einen Alkalimetallverbindung vermischt werden und das daraus erhaltene Gemisch anschließend mit der oxidischen Wolframverbindung zu einer Katalysatormasse vermischt werden.

Die oxidische Wolframverbindung wird bevorzugt als Feststoff zu dem Trägermaterial bzw. dem Gemisch aus Trägermaterial und der mindestens einen Alkalimetallverbindung gegeben. Besonders bevorzugt ist hierbei die Zugabe fester Wolframsäure vor oder nach der Zugabe einer wässrigen Lösung eines Alkalimetallhydroxids mit einer Konzentration an Alkalimetallhydroxid, bezogen auf das Gesamtgewicht der Lösung, von ≥ 50 Gew.-%. Besonders bevorzugt wird feste Wolframsäure vor oder nach einer 70%igen wässrigen Alkalimetallhydroxidlösung zu dem Trägermaterial gegeben.

Das Formen des Katalysators erfolgt bevorzugt durch Extrusion der erhaltenen Katalysatormasse oder Pressen derselben, beispielsweise in einer Tablettenpresse. Bei der Extrusion sollte die Temperatur bevorzugt 40 °C nicht übersteigen. Vorzugsweise beträgt der Druck hierbei mindestens 5 bar.

Bei dem nach dem erfindungsgemäßen Verfahren hergestellten Katalysator handelt es sich bevorzugt um einen Vollkatalysator, d. h. einen Katalysator, bei welchem die Katalysatormasse homogen über den gesamten Katalysatorformkörper, der bevorzugt in Form von Extrudaten oder Presslingen vorliegt, verteilt ist, im Gegensatz zu einem Schalenkatalysator, bei welchem eine Mischung, enthaltend die oxidische Zusammensetzung, auf einen Trägerkern in Form einer Schale aufgebracht wird.

Das erfindungsgemäße Verfahren dient bevorzugt zur Herstellung von Katalysatoren zur Verwendung in einem Festbettreaktor, welche bevorzugt in Form von diskreten Katalysatorteilchen vorliegen. Diese Katalysatorteilchen haben bevorzugt eine Größe im Bereich von 1 bis 9 mm, besonders bevorzugt von 1,5 bis 5 mm.

Die geometrische Form dieser Katalysatorteilchen ist grundsätzlich nicht beschränkt. Die Katalysatorteilchen können beispielsweise in Form von Kugeln, Zylindern, Quadern, Würfeln, Prismen, Ellipsoiden, Ringen oder auch unregelmäßig geformten Teilchen vorliegen, ohne auf diese Formen beschränkt zu sein.

Die Schüttdichte des so erhaltenen Katalysatormaterials beträgt bevorzugt mehr als 0,70 g/cm³, weiterhin bevorzugt mehr als 0,80 g/cm³, besonders bevorzugt mehr als 0,9 g/cm³ und insbesondere 1,0 bis 1,7 g/cm³ (bestimmt gemäß DIN ISO 697).

Die spezifische BET-Oberfläche des Katalysators kann weniger als 180 m²/g betragen, ermittelt nach ISO 9277, und sogar weniger als 90 m²/g, beträgt jedenfalls aber mehr als 10 m²/g. Bevorzugt beträgt sie 30-80 m²/g, besonders bevorzugt 40-60 m²/g.

Dies ist umso überraschender, als da auch bei diesen vergleichsweise geringen Werten für die BET-Oberfläche sehr gute Ergebnisse erzielt werden, während die BET-Oberfläche der aus dem Stand der Technik bekannten Katalysatoren im Allgemeinen 140 und mehr g/m² beträgt und bei diesen umso bessere Ergebnisse erzielt werden, je höher die BET-Oberfläche ist.

Das spezifische Porenvolumen der Mesoporen des Katalysators, d. h. der Poren mit einem Durchmesser im Bereich von 2 bis 50 nm, beträgt vorzugsweise weniger als 0,20 ml/g, besonders bevorzugt weniger 0,15 ml/g, bestimmt nach Barrett, Joyner und Halenda (BJH) gemäß DIN 66134.

Das spezifische Porenvolumen der Makroporen des Katalysators, d. h. der Poren mit einem Durchmesser oberhalb von 50 nm, beträgt bevorzugt weniger als 0,40 ml/g, besonders bevorzugt weniger als 0,25 ml/g, bestimmt durch Quecksilberintrusion gemäß DIN 66133.

Das erfindungsgemäße Verfahren zur Herstellung des Katalysators umfasst ferner bevorzugt wenigstens einen, bevorzugt beide der folgenden zusätzlichen Schritte: (i) Trocknen der Katalysatormasse und/oder des geformten Katalysators und (ii) Calcinieren der Katalysatormasse und/oder des geformten Katalysators.

Das Trocknen und Calcinieren kann zum einen an der noch nicht zum Katalysatorteilchen geformten Katalysatormasse vorgenommen werden, also nach Schritt 2 und vor Schritt 3 des erfindungsgemäßen Verfahrens. Dies ist bevorzugt der Fall, wenn die Katalysatormasse durch Pressen geformt werden soll. Das Trocknen und Calcinieren kann auch an den bereits geformten Katalysatorteilchen vorgenommen werden, also nach Schritt 3 des erfindungsgemäßen Verfahrens. Letzteres ist bevorzugt der Fall, wenn die Formgebung der Katalysatormasse durch Extrusion erfolgt.

Weiterhin kann die Katalysatormasse bzw. der geformte Katalysator auch für eine Dauer von etwa 1 bis 10 Stunden bei Raumtemperatur vorgetrocknet werden.

Die eigentliche Trocknung erfolgt bevorzugt für eine Dauer von 1 bis 10 Stunden bei einer Temperatur von 100 bis 200 °C, besonders bevorzugt bei einer Temperatur von 100 bis 140 °C. Die Calcinierung erfolgt bevorzugt für eine Dauer von 1 bis 20 Stunden, bevorzugt von 1 bis 10 Stunden und besonders bevorzugt von 1 bis 5 Stunden bei einer Temperatur von 300 bis 600 °C, besonders bevorzugt bei einer Temperatur von 420 bis 480 °C. Sowohl die Trocknung als auch die Calcinierung können beispielsweise in einem Muffelofen erfolgen, wobei sich die Calcinierung auch unmittelbar an die Trocknung anschließen kann, ohne dass der Katalysator zwischendurch abgekühlt wird. Optional kann die Schüttung des Katalysators bei der Vortrocknung, der Trocknung und/oder der Calcinierung auch von einem Gasstrom durchströmt werden, der den Abtransport der Restfeuchte erleichtert. Geeignete Gase sind beispielsweise Luft, Kohlendioxid, Stickstoff, Edelgase etc., ohne hierauf beschränkt zu sein.

In dem erfindungsgemäßen Verfahren können der Katalysatormasse ferner in einem der zuvor beschriebenen Schritte des Vermischens einzelner Bestandteile der Katalysatormasse (Schritt 2) oder des Formens des Katalysators (Schritt 3) weitere Stoffe hinzugesetzt werden wie beispielsweise Lösungsmittel, Bindemittel, Pressmittel, Porenbildner, mineralische Fasern oder Gleitmittel. Bevorzugt werden in einem oder beiden der oben genannten Schritte 2 und 3 mindestens ein organisches und/oder ein anorganisches Bindemittel und/oder mineralische Fasern dem Gemisch aus Trägermaterial mit Wolframsäure und/oder Alkalimetallverbindung hinzugesetzt. Geeignete Bindemittel umfassen beispielsweise kolloidale Kieselsäuredispersionen, hochpolymere Polysaccharide wie beispielsweise Hydroxyethylcellulose oder Methylhydroxyethylcellulose und Wachsdispersionen, ohne auf diese beschränkt zu sein.

Bevorzugt wird als Bindemittel mindestens eine kolloidale Kieselsäuredispersion verwendet, besonders bevorzugt in Kombination mit organischen Bindemitteln. Der Anteil des Bindemittels bzw. der Bindemittel, sofern mehrere verwendet werden, in der erfindungsgemäßen Zusammensetzung beträgt bevorzugt 0,1 bis 20 Gew.-%, bezogen auf die Gesamtzusammensetzung, besonders bevorzugt 1 bis 10 Gew.-%. Wird eine kolloidale Kieselsäuredispersion als Bindemittel verwendet, so beträgt der Anteil dieses Bindemittels in dem getrockneten, calcinierten und geformten Katalysator bevorzugt 3 bis 7 % und insbesondere 4 bis 6 %, bezogen auf den SiO₂-Gehalt in der gesamten Trägermasse.

Der Anteil des Trägermaterials in den erfindungsgemäßen Katalysatorteilchen beträgt bevorzugt 20 bis 85 Gew.-%, besonders bevorzugt 25 bis 50 Gew.-%, bezogen auf die Gesamtzusammensetzung.

Die vorliegende Erfindung betrifft ferner einen Katalysator zur Herstellung von Alkylmercaptanen durch Umsetzung von Alkanolen mit Schwefelwasserstoff, welche ein Trägermaterial und eine oxidische Zusammensetzung, enthaltend neben Sauerstoff mindestens ein Alkalimetall und Wolfram, umfasst und der hergestellt ist aus Partikeln des Trägermaterials mit einer Partikelgröße von weniger als 1000 µm, bevorzugt weniger als 500 µm, weiterhin bevorzugt weniger als 250 µm, besonders bevorzugt weniger als 100 µm und insbesondere aus Partikeln mit einer Partikelgröße im Bereich von 1 bis 25 µm.

Der Katalysator ist bevorzugt ein Katalysator, in welchem die Standardabweichung Stabw der normierten Wolframkonzentration (Cₙₒᵣₘ (W)) über den Querschnitt des Katalysators weniger als 20,0 beträgt, ermittelt durch quantitative EDX-Messung gemäß ISO 22309 (2006) in quadratischen Messpunkten mit jeweils 100 µm Seitenlänge, deren Mittelpunkte auf einer Gerade liegen und jeweils 100 µm vom jeweils angrenzenden Quadratmittelpunkt entfernt sind, wobei der jeweils erste und letzte Quadratmittelpunkt jeweils etwa 100 µm vom Rand des vermessenen Katalysatorquerschnitts entfernt ist. Im Sinne der Erfindung stellt die normierte Konzentration Cₙₒᵣₘ den Quotienten aus der experimentell bestimmten Konzentration des betreffenden Elements im jeweiligen Quadrat und dem Mittelwert der Konzentration dieses Elements über den Querschnitt der Probe, d. h. die Summe aller quadratischen Messpunkte, multipliziert mit dem Faktor 100 dar.

Die Anzahl und Lage der Messpunkte wird einerseits in gewissem Maße durch die Größe und Form des Katalysatorteilchens bestimmt, die Anzahl beträgt bevorzugt jedoch mindestens 15, weiterhin bevorzugt mindestens 20, besonders bevorzugt mindestens 25 und insbesondere mindestens 30 und bevorzugt höchstens 200, weiterhin bevorzugt höchstens 150, besonders bevorzugt höchstens 100 und insbesondere höchstens 50. Ganz besonders bevorzugt kann beispielsweise ein Wert von 40 +/- 2 Messpunkten sein. Bei einem kugelförmigen Katalysatorteilchen verläuft die durch die Mittelpunkte der Messquadrate definierte Gerade bevorzugt durch den Mittelpunkt der Kugel, wobei ihre Länge dem Kugeldurchmesser entspricht. Bei einem von einer Kugelform abweichend geformeten Katalysatorteilchen, bspw. einem Extrudat, schneidet die durch die Mittelpunkte der Messquadrate definierte Gerade bevorzugt wenigstens die Längsachse des Teilchens, besonders bevorzugt im geometrischen Schwerpunkt des Teilchens, wobei die Länge dieser Geraden mindestens die Länge des Querschnittdurchmessers aufweist. Die durch die Mittelpunkte der Messquadrate definierte Gerade kann aber auch mit der Längsachse identisch sein.

Bevorzugt beträgt die Mittelabweichung, d. h. die durchschnittliche absolute Abweichung vom Mittelwert, der auf diese Weise ermittelten Wolframkonzentration weniger als 15,0 und/oder die Differenz zwischen dem Maximalwert der ermittelten normierten Wolframkonzentration und dem Minimalwert Δ (Max - Min) weniger als 90,0.

Die Standardabweichung der auf diese Weise ermittelten Cäsiumkonzentration beträgt bevorzugt weniger als 15,5 und die Differenz zwischen dem Maximum und dem Minimum der normierten Cäsiumkonzentration weniger als 64,0.

Die Ermittlung der zuvor genannten Parameter wird in den nachfolgenden Beispielen veranschaulicht.

Die oxidische Zusammensetzung ist bevorzugt eine Zusammensetzung der allgemeinen Formel AₓWO_{y}, in welcher bedeuten
A: mindestens ein Alkalimetall, bevorzugt ausgewählt aus der Gruppe, bestehend aus Natrium, Kalium, Rubidium und Cäsium,
x: molarer Anteil des mindestens einen Alkalimetalls im Verhältnis zu Wolfram in der Zusammensetzung, welches bevorzugt im Bereich von 2:1 zu 0,8:1 liegt, besonders bevorzugt im Bereiche von 1,9:1 zu 1,1:1, und
y: molarer Anteil Sauerstoff in der Zusammensetzung, welcher bevorzugt im Bereich von 3,4 bis 4 liegt.

Die oxidische Zusammensetzung in dem Katalysator entspricht der bereits oben für das erfindungsgemäße Verfahren definierten.

Bevorzugt ist der erfindungsgemäße Katalysator hergestellt durch ein Verfahren, welches einen Schritt des Vermengens einer festen Wolframverbindung, bevorzugt einer festen oxidischen Wolframverbindung, mit dem Trägermaterial umfasst, wie dies bereits oben beschrieben wurde. Bei der oxidischen Wolframverbindung handelt es sich bevorzugt um die bereits oben beschriebenen Wolframverbindungen.

Der Anteil der oxidischen Zusammensetzung aus Alkalimetall und Wolfram in dem Katalysator beträgt bevorzugt mehr als 15 Gew.-%, weiterhin bevorzugt mehr als 25 Gew.-%, weiterhin bevorzugt mehr als 36 Gew.-%, besonders bevorzugt mehr als 40 Gew.-% und insbesondere mehr als 45 Gew.-%, bezogen auf das Gesamtgewicht des Katalysators. Bevorzugt beträgt der Anteil der oxidischen Zusammensetzung in dem Katalysator bis zu 60 Gew.-%.

Bei dem Trägermaterial handelt es sich bevorzugt um mindestens eine oxidische anorganische Verbindung, welche besonders bevorzugt ausgewählt ist aus der Gruppe enthaltend Aluminiumoxid, Siliciumoxid, Titandioxid, Zirconoxid, amorphe Aluminiumsilikate und Gemische derselben. Bevorzugte Ausgestaltung dieses Trägermaterials sind bereits für das erfindungsgemäße Verfahren beschrieben worden.

Der erfindungsgemäße Katalysator umfasst ferner bevorzugt mindestens ein organisches und/oder anorganisches Bindemittel, wobei diese Bindemittel bevorzugt eines der oben bereits genannten Bindemittel darstellen und bevorzugt in den bereits genannten Gewichtsanteilen in der Katalysatorzusammensetzung vorhanden sind.

Der erfindungsgemäße Katalysator ist bevorzugt hergestellt nach dem bereits beschriebenen erfindungsgemäßen Verfahren zur Herstellung eines Katalysators. Ferner betrifft die Erfindung ein Verfahren zur Herstellung von Alkylmercaptanen durch Umsetzung von Alkanolen mit Schwefelwasserstoff, bevorzugt zur Herstellung von Methylmercaptan durch Umsetzung von Methanol mit Schwefelwasserstoff, in Gegenwart des erfindungsgemäßen Katalysators bzw. eines Katalysators, welcher nach dem erfindungsgemäßen Verfahren zur Herstellung eines Katalysators hergestellt wurde.

Dieses Verfahren wird bevorzugt bei einer Temperatur von 250 bis 500 °C, besonders bevorzugt von 300 bis 400 °C durchgeführt. Das Massenverhältnis von Schwefelwasserstoff zu Methanol liegt bevorzugt im Bereich von 1:1 1 bis 10:1, weiterhin bevorzugt im Bereich von 1:1 1 bis 5:1, besonders bevorzugt im Bereich von 1:1 bis ≤ 3:1 und insbesondere im Bereich von 1:1 bis ≤ 2:1. Die Reaktion wird hierbei bevorzugt bei einem Druck im Bereich von 1 bis 20 bar, besonders bevorzugt im Bereich von 3 bis 10 bar durchgeführt. Die Reaktion wird bevorzugt bei einem Alkanolumsatz von ≥ 90%, weiterhin bevorzugt ≥ 92,5% besonders bevorzugt ≥ 95% und insbesondere ≥ 98 % oder sogar ≥ 99 % durchgeführt.

### Verzeichnis der Abbildungen

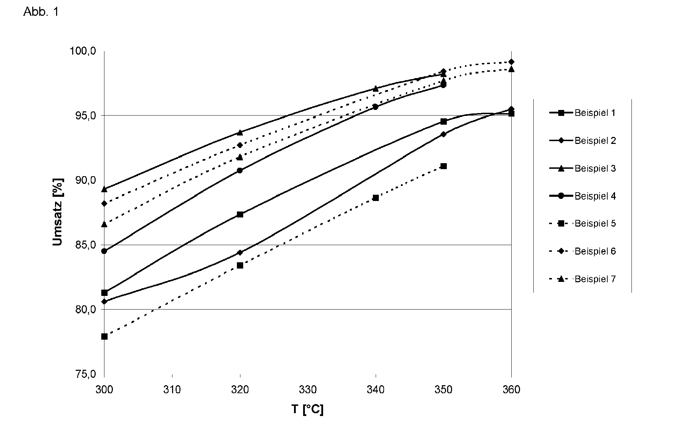

Abbildung 1 zeigt den mit erfindungsgemäßen Katalysatoren (Beispiele 3 bis 7) erzielten Umsatz bei der Reaktion von Schwefelwasserstoff und Methanol zu Methylmercaptan bei verschiedenen Temperaturen unter den in Beispiel 8 beschriebenen Bedingungen im Vergleich zu demjenigen mit Katalysatoren, die nach aus dem Stand der Technik bekannten Verfahren hergestellt wurden (Vergleichsbeispiele 1 und 2).

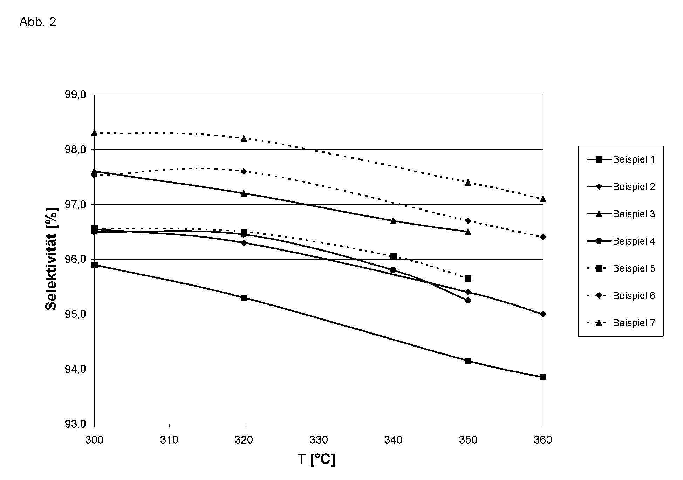

Abbildung 2 zeigt die mit erfindungsgemäßen Katalysatoren (Beispiele 3 bis 7) erzielte Selektivität bei der Reaktion von Schwefelwasserstoff und Methanol zu Methylmercaptan bei verschiedenen Temperaturen unter den in Beispiel 8 beschriebenen Bedingungen im Vergleich zu derjenigen mit Katalysatoren, die nach aus dem Stand der Technik bekannten Verfahren hergestellt wurden (Vergleichsbeispiele 1 und 2).

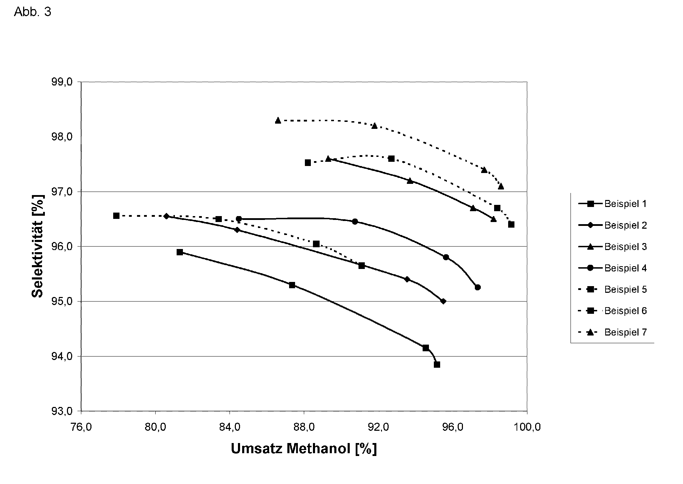

Abbildung 3 verdeutlicht den Zusammenhang zwischen Umsatz und Selektivität für die Katalysatoren der Beispiele 1 bis 7.

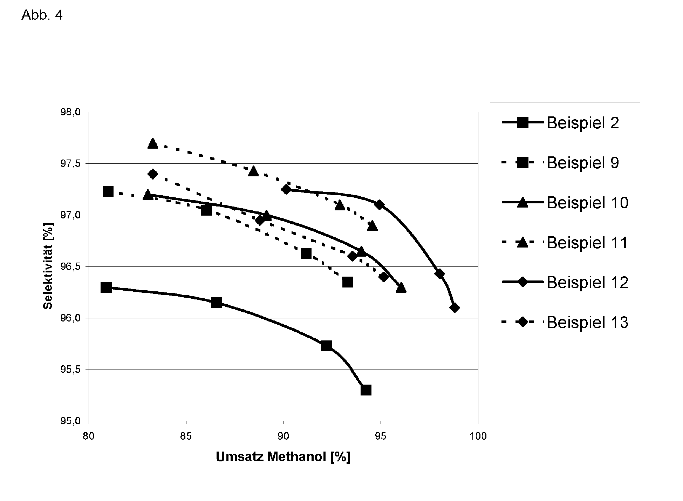

Abbildung 4 zeigt den Zusammenhang zwischen Umsatz und Selektivität bei erfindungsgemäßen Katalysatoren unterschiedlicher Beladung (Beispiele 9 bis 13) bei der Reaktion von Schwefelwasserstoff und Methanol zu Methylmercaptan unter den in Beispiel 8 beschriebenen Bedingungen im Vergleich zu einem Katalysator, der nach einem aus dem Stand der Technik bekannten Verfahren hergestellt wurde (Vergleichsbeispiel 2).

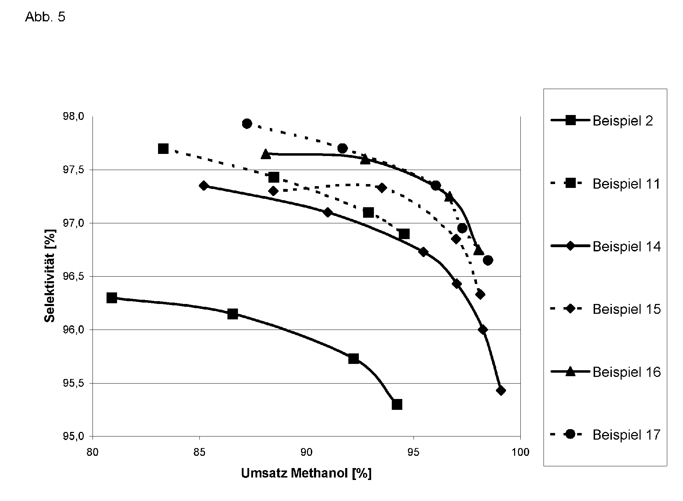

Abbildung 5 zeigt den Zusammenhang zwischen Umsatz und Selektivität für verschiedene erfindungsgemäße Katalysatoren gleicher Beladung (Beispiele 11 sowie 14 bis 17), bei welchen die Katalysatormasse auf unterschiedlichem Wege hergestellt wurde bzw. die Formgebung in unterschiedlichen Verfahren erfolgte, im Vergleich zu einem aus dem Stand der Technik bekannten Katalysator (Vergleichsbeispiel 2) bei der Reaktion von Schwefelwasserstoff und Methanol zu Methylmercaptan.

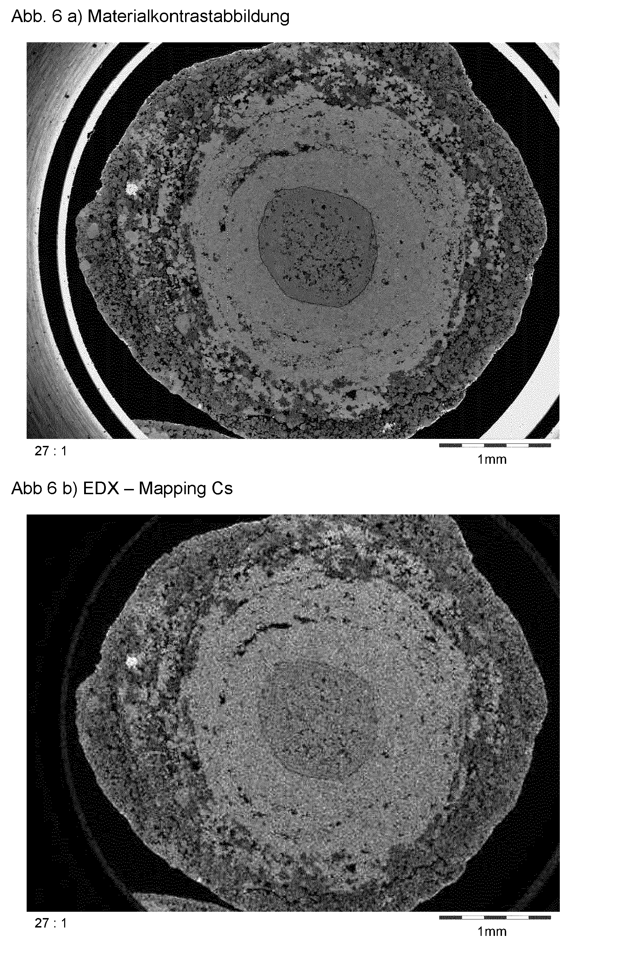

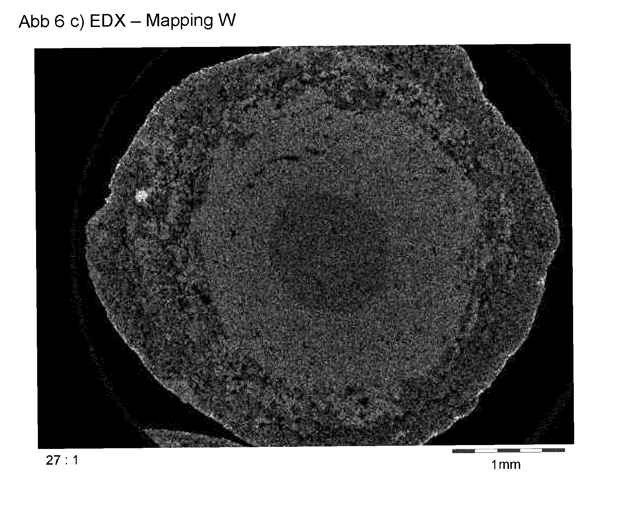

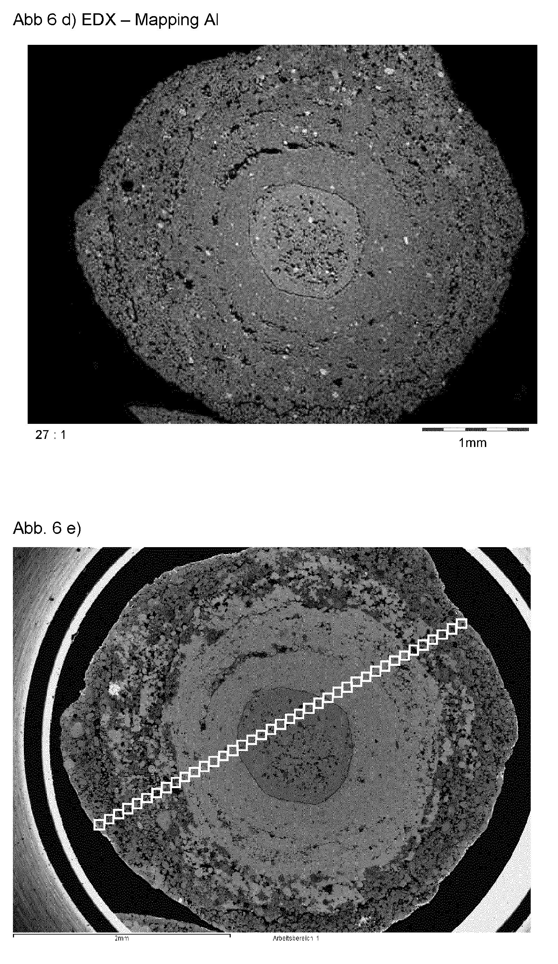

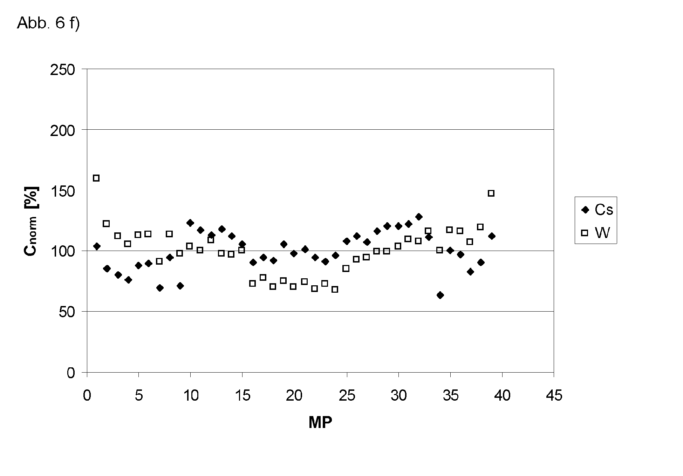

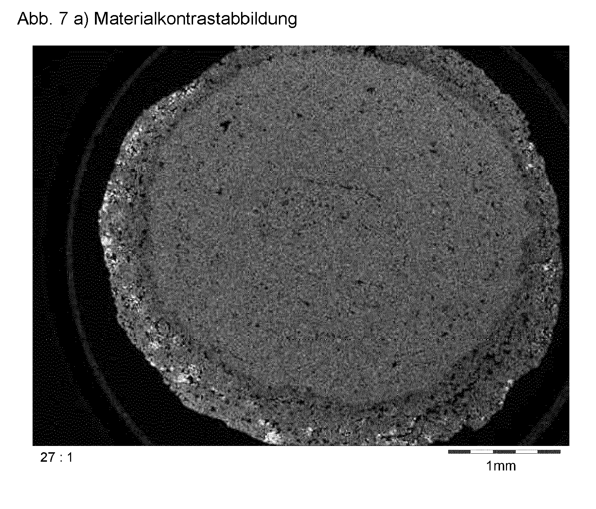

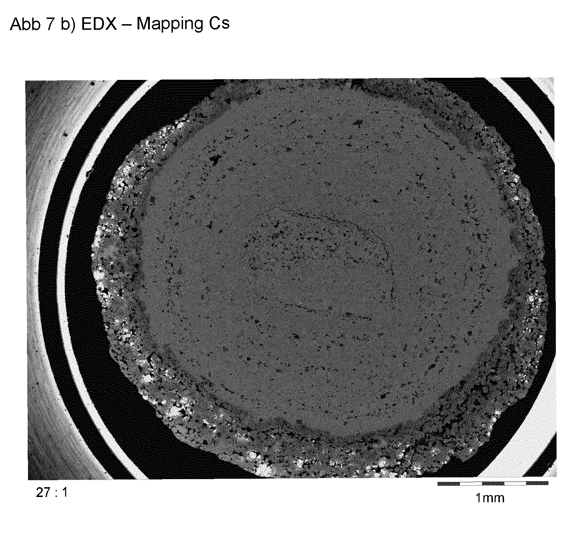

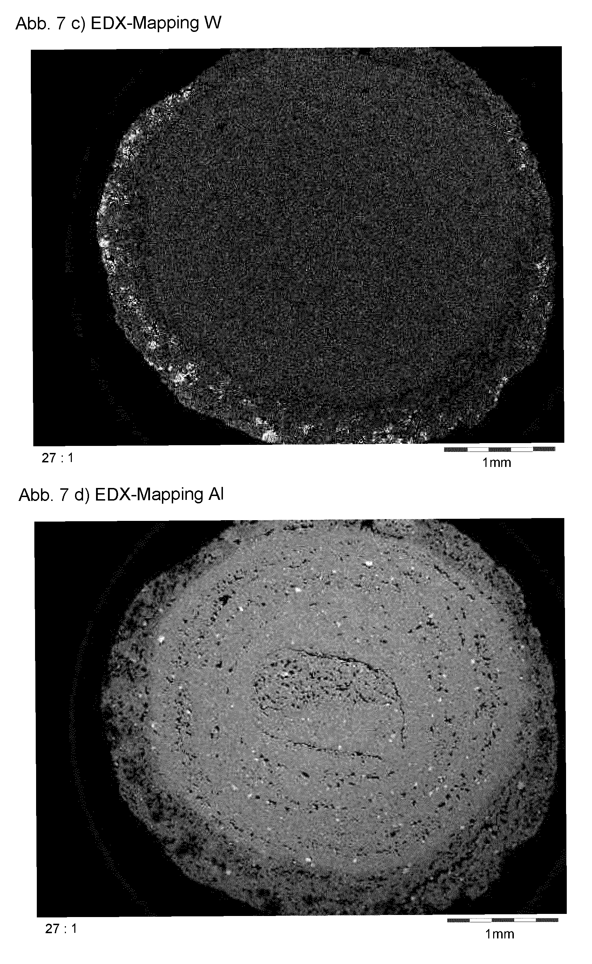

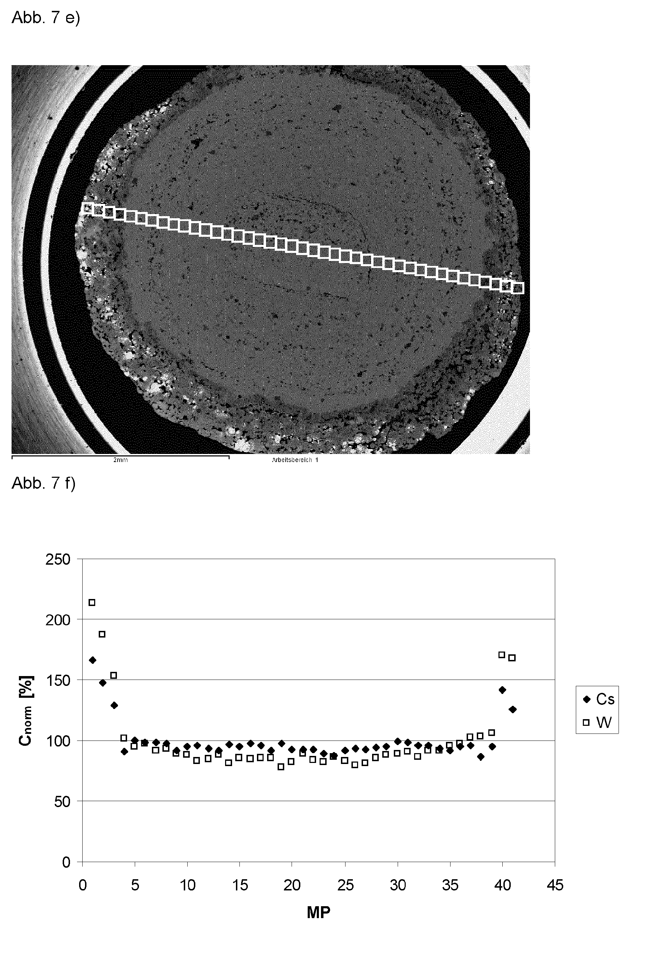

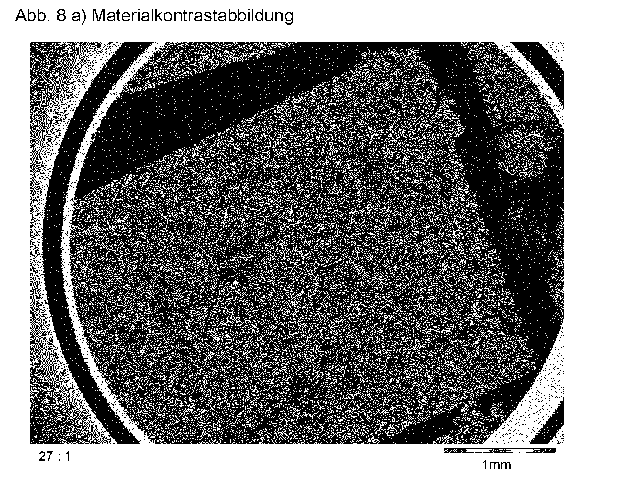

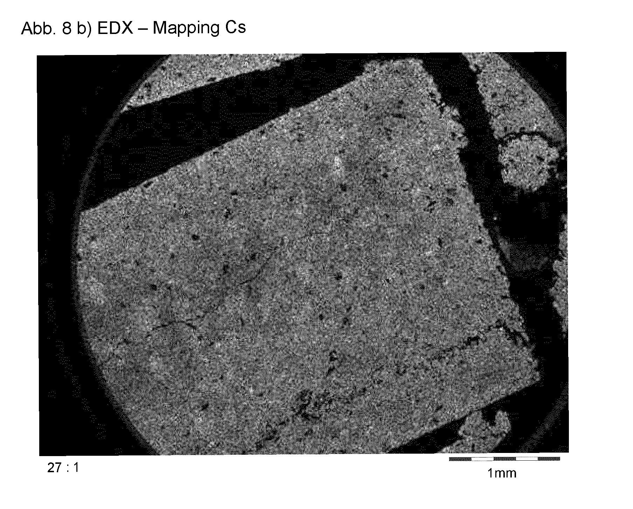

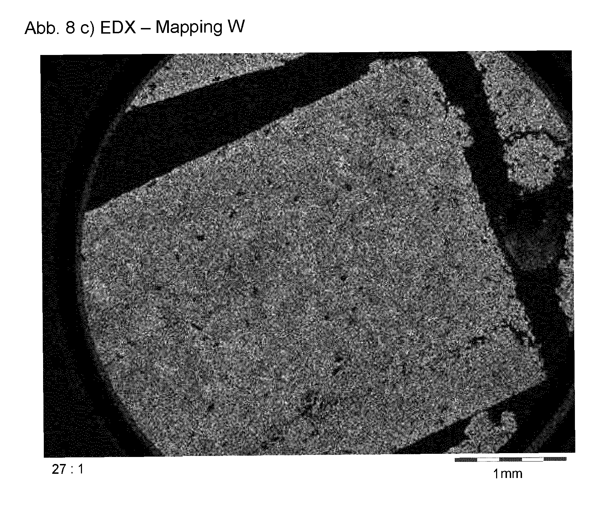

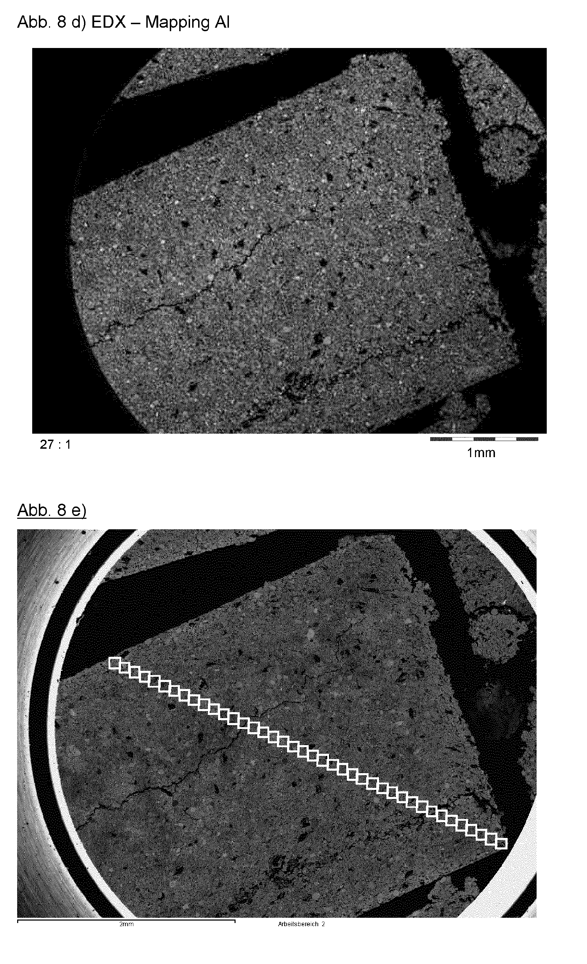

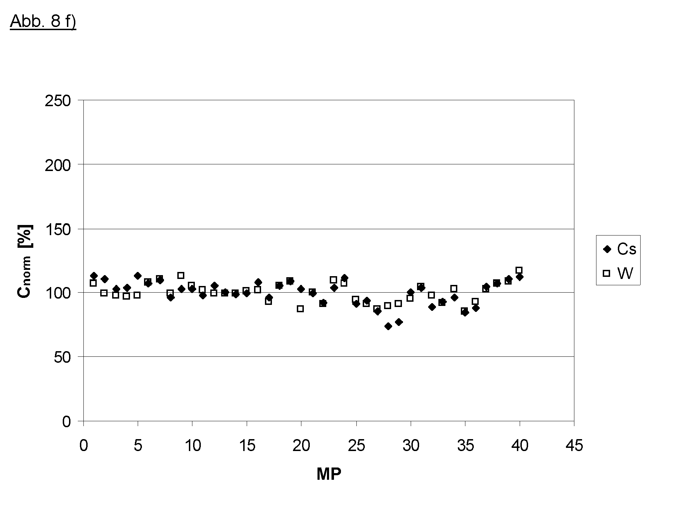

Die Abbildungen 6 bis 8 zeigen die Oberflächenverteilung der Elemente Cäsium, Wolfram und Aluminium in zwei Katalysatoren, die nach aus dem Stand der Technik bekannten Verfahren hergestellt wurden (Abbildung 6: Beispiel 1; Abbildung 7: Beispiel 2) im Vergleich zu einem erfindungsgemäßen Katalysator (Abbildung 8: Beispiel 6), ermittelt durch EDX-Mapping. Als jeweilige Abbildung a) ist eine Materialkontrastabbildung eines Katalysatorteilchens gezeigt, in welcher bereits die weitgehende Homogenität (Abb. 8a: Beispiel 6) bzw. Inhomogenität (Abb. 6a) der Katalysatorstruktur bzw. der Verteilung der Elemente in demselben erkennbar wird. Die jeweiligen Abbildungen b) ― d) zeigen die Konzentration der Elemente Cäsium (Abb. b), Wolfram (Abb. c) und Aluminium (Abb. d) in dem Katalysatorpartikel. Unter der jeweiligen Abbildung ist auch eine Skala abgebildet, welche die Länge von einem Millimeter der Originalstruktur maßstabsgerecht verdeutlichen soll. Die jeweilige Abbildung e) zeigt die Verteilung der Messquadrate zur Quantifizierung der Homogenität der Verteilung der Elemente Cäsium und Wolfram über den Katalysatorquerschnitt, wie dies in Beispiel 18 näher beschrieben ist; die jeweilige Abbildung f) zeigt die grafische Auswertung dieser Messungen.

### Beispiele

### Beispiel 1 (Vergleichsbeispiel):

200 g kugelförmiges Aluminiumoxid mit einem Partikeldurchmesser von 2 bis 5 mm (Spheralite 501 A der Firma Axens mit einer spezifischen Oberfläche von 303 m²/g, einem Porenvolumen von 45 mL/100 g und einer Schüttdichte von 815 kg/m³) wurden in einer dreistufigen Imprägnierung mit insgesamt 52.8 Gew.-% oxidischer Zusammensetzung der Formel Cs_{1.44}WO_{3.72} mithilfe der Vakuumimprägnierung imprägniert. Hierzu wurde wie folgt vorgegangen: 103,3 g Wolframsäure wurden in 206,5 g 32%iger Ammoniaklösung suspendiert und unter Rühren für etwa 30 Minuten aufgelöst. 126,9 g einer 70%igen Cäsiumhydroxidlösung in Wasser wurden zu dieser Lösung hinzugegeben, und die resultierende Lösung wurde für etwa 23 bis 24 Stunden gerührt. Das Aluminiumoxid wurde in einem Glasgefäß vorgelegt, das auf 150 mbar evakuiert worden war. Durch Öffnen eines Hahns wurde die Imprägnierlösung solange angesaugt, bis über dem gesamten Aluminiumoxid ein Überstand an Imprägnierlösung von etwa 4,5 cm bestand. Nach Belüftung des Glasgefäßes wurde der Träger in der Lösung für etwa 15 Minuten inkubiert. Anschließend wurde die Lösung abgelassen, und der Katalysator wurde durch Hindurchleiten eines Luftstroms von 200 NI/h (Volumenstrom im Normzustand bei 0 °C und 1,013 bar absolut nach DIN 1343) für etwa 1 Stunde vorgetrocknet, wobei noch anhaftende Imprägnierlösung in den Vorlagenbehälter gespült wurde.

Anschließend wurde der Katalysator unter einem Luftstrom von 60 m³/h mit einer Aufheizrate von 1 °C/min auf 120 °C erwärmt und drei Stunden bei dieser Temperatur gehalten. Danach wurde die Temperatur mit einer Aufheizrate von 5 °C/min auf 455 °C erhöht, und der Katalysator 3 Stunden bei dieser Temperatur calciniert.

Zur Durchführung der zweiten Imprägnierung wurde eine bereits für den ersten Schritt beschriebene Imprägnierlösung angesetzt und in gleicher Weise durch Vakuumimprägnierung auf den bereits beladenen Katalysator, welcher aus der ersten Imprägnierung erhalten wurde, aufgebracht. Die Vortrocknung bei Raumtemperatur, gefolgt von der dreistündigen Trocknung bei 120 °C und der anschließenden Calcinierung bei 455 °C für 3 Stunden erfolgte wie bereits beschrieben.

Zur Durchführung der dritten Imprägnierung wurde ebenso verfahren.

### Beispiel 2 (Vergleichsbeispiel):

Vergleichsbeispiel 1 wurde mit einer Beladung des Aluminiumoxids von 17,8 Gew.-% WO₃ und 17,3 Gew.-% Cs₂O wiederholt.

### Beispiel 3:

In diesem erfindungsgemäßen Beispiel wurden 80 g eines pulverförmigen Aluminiumoxids mit einem Partikeldurchmesser von 7 bis 15 µm (Spheralite 509A) der Firma Axens mit einer spezifischen Oberfläche von 335 m²/g, einem Porenvolumen von 56 mL/100 g und einer Schüttdichte von 840 kg/m³) nacheinander mit fester Wolframsäure und einer Calciumhydroxidlösung vermischt.

Hierzu wurde wie folgt vorgegangen:
In einem Becherglas wurden 80 g des Aluminiumoxids mit 40,98 g fester Wolframsäure vermischt. Zu dem pulvrigen Gemisch wurden 69,85 g einer 50%igen Cäsiumhydroxidlösung in Wasser und 5,33 g einer 6%igen wässrigen Methylhydroxyethylcelluloselösung (Tylose MH 1000, ShinEtsu, Tokio, Japan) vermischt und 10 Minuten unter Zuhilfenahme eines Spatels verknetet, bis eine extrudierbare Masse entstand, d. h. die Flüssigkeit komplett aufgenommen und eine teigartige, nicht klebrige Masse erhalten wurde (ca. 10 min). 5,33 g Petroleum (Merck, Darmstadt, Deutschland) wurden hinzugegeben und mit dem Gemisch verknetet. In einem Muffelofen wurde das Gemisch getrocknet und anschließend calciniert, indem es zunächst mit einer Aufheizrate von 2 °C/min auf 120 °C aufgeheizt wurde, 3 Stunden bei dieser Temperatur gehalten wurde, dann mit einer Aufheizrate von 5 °C/min auf 455 °C erwärmt wurde und 3 Stunden bei dieser Temperatur gehalten wurde. Anschließend wurde das Gemisch auf 20 °C abgekühlt.

Das erhaltene Granulat wurde nach dem Abkühlen in einem Mörser gemahlen. Jeweils 1 bis 2 g des erhaltenen Katalysatorpulvers wurden anschließend in einer Tablettenpresse bei einem Druck von 4 t für ca. 1 Minute zu einer Tablette mit 20 mm Durchmesser gepresst.

Für den nachfolgenden Einsatz in einem Testreaktor zur Herstellung von Methylmercaptan wurde die Tablette in Stücke von max. 5 mm Kantenlänge zerkleinert.

### Beispiel 4:

Beispiel 3 wurde mit einer Beladung des Aluminiumoxids von 17,8 % WO₃ und 17,3 Gew.-% Cs₂O wiederholt.

### Beispiel 5:

Beispiel 4 wurde wiederholt, wobei statt des pulverförmigen Aluminiumoxids mit einem Partikeldurchmesser von 7 bis 15 µm (Spheralite 509 A) Aluminiumoxid mit einem Partikeldurchmesser von weniger als 250 µm verwendet wurde, das durch Mahlen des kugelförmigen Aluminiumoxids mit einem Partikeldurchmesser von 2 bis 5 mm (Spheralite 501 A) gewonnen wurde.

### Beispiel 6: Herstellung der erfindungsgemäßen Katalysatorteilchen unter Zusatz von Bindern und Formgebung durch Extrusion

1,05 kg Spheralite 509 A und 537,9 g Wolframsäure wurden in einem Laborchargenkneter (Coperion LUK 2.5, Weinheim, Stuttgart, Deutschland) bei 40 Umdrehungen/min der Knethaken und 11 Umdrehungen/min der Austragsschnecke (rückwärtsgerichtet) gemischt, wobei der in dem Kneter befindliche Trog mit einem Kryostat auf 10 °C gekühlt wurde. Anschließend wurden 740,5 g einer 70 Gew.-%igen wässrigen Cäsiumhydroxidlösung innerhalb 1 min unter ständigem Durchmischen hinzugegeben, wobei die Temperatur kurzzeitig um 30 bis 40 °C stieg. 10 Minuten nach beendeter Zugabe wurden 127,5 g vollentsalztes Wasser und anschließend 175 g einer kolloidalen Kieselsäuredispersion (Lithosol 1530, Zschimmer & Schwarz GmbH & Co. KG, Lahnstein, Deutschland) hinzugegeben. Das erhaltene Gemisch wurde für weitere 10 Minuten durchmischt, bevor eine Mischung von 30 g eines hochpolymeren Polysaccharids (Zusoplast PS 1, Zschimmer & Schwarz GmbH & Co. KG, Lahnstein, Deutschland) und 30 g Hydroxyethylcellulose (Tylose H 10000 P2 ShinEtsu, Tokio, Japan) hinzugegeben wurden. Die Binder wurden unter ständigem Kneten der Masse für 120 Minuten quellen gelassen. Anschließend wurden 15 g einer nichtionischen Wachsdispersion (Zusoplast WE8, Zschimmer & Schwarz GmbH & Co. KG, Lahnstein, Deutschland) hinzugefügt. Nach einer Gesamtknetzeit von 190 Minuten wurde mit der Extrusion begonnen, wozu bei gleichbleibender Kneterdrehzahl die Drehrichtung der Schnecke auf Fördern umgestellt wurde und die Drehzahl der Schnecke auf 13 Umdrehungen/min erhöht wurde. Als Presswerkzeug wurde ein Aufsatz mit vier horizontalen Bohrungen mit einem jeweiligen Durchmesser von 3,2 mm verwendet. Es wurden zwei horizontal schneidende Schneiddrähte mit 400 Umdrehungen/min betrieben, um Extrudate einer Länge von etwa 3,2 mm zu erhalten. Der Drüsendruck betrug 12,7 bar. Die geschnittenen Extrudate wurden auf ein Trockenband fallen gelassen und bei 60 °C vorgetrocknet, bevor sie in einem Muffelofen mit einer Aufheizrate von 1 °C/min auf 120 °C erwärmt wurden und 3 Stunden bei Temperatur gehalten wurden. Zum Calcinieren wurden die Extrudate direkt anschließend mit einer Aufheizrate von 5 °C/min auf 455 °C erwärmt und 3 Stunden bei dieser Temperatur gehalten.

### Beispiel 7:

Beispiel 6 wurde wiederholt, wobei dieses Mal die Zugabe der Cäsiumhydroxidlösung vor der Zugabe der festen Wolframsäure erfolgte.

### 1.1. Beispiel 8: Anwendungsbeispiel

Die in den Beispielen 1 bis 7 hergestellten Katalysatoren wurden hinsichtlich ihrer Leistungscharakteristika bei der Synthese von Methylmercaptan aus Schwefelwasserstoff und Methanol untersucht.

Die Umsetzung von Schwefelwasserstoff und Methanol zu Methylmercaptan in Gegenwart des entsprechenden Katalysators wurde in einem Edelstahlrohr von 18 mm Durchmesser und einer Länge von 500 mm durchgeführt. Es wurde jeweils eine Katalysatorschüttung eines Volumens von 76 mL eingesetzt, welche jeweils beiderseits durch Inertschüttungen aus Glaskugeln im Reaktionsrohr fixiert wurde. Das Reaktionsrohr wurde über einen Doppelmantel mit einem Thermoöl auf die unterschiedlichen, in der nachfolgenden Tabelle 1 angegebenen Reaktionstemperaturen im Bereich von 300 bis 360 °C erwärmt.

**Tabelle 1**

| **Katalysator** | **T** | **Umsatz** | **Selektivität** | **Träger*** | **Beladung** | |
|---|---|---|---|---|---|---|
| | [°C] | [%] | [%] | | WO₃ [Gew.-%] | Cs₂O [Gew.-%] |
| **Beispiel 1 (Vergleichsbeispiel)** | 300 | 81,3 | 95,9 | I (imp) | 28,20 | 24,6 |
| | 320 | 87,4 | 95,3 | | | |
| | 350 | 94,6 | 94,2 | | | |
| | 360 | 95,2 | 93,9 | | | |
| **Beispiel 2** (Vergleichsbeispiel) | 300 | 80,6 | 96,6 | I (imp) | 17,80 | 17,3 |
| | 320 | 84,4 | 96,3 | | | |
| | 350 | 93,6 | 95,4 | | | |
| | 360 | 95,5 | 95,0 | | | |
| **Beispiel 3** | 300 | 89,3 | 97,6 | II (press 4) | 25,81 | 22,49 |
| | 320 | 93,7 | 97,2 | | | |
| | 340 | 97,1 | 96,7 | | | |
| | 350 | 98,2 | 96,5 | | | |
| **Beispiel 4** | 300 | 84,5 | 96,5 | II (press 4) | 17,80 | 17,3 |
| | 320 | 90,8 | 96,5 | | | |
| | 340 | 95,7 | 95,8 | | | |
| | 350 | 97,4 | 95,3 | | | |
| **Beispiel 5** | 300 | 77,9 | 96,6 | I (mill, press 4) | 17,80 | 17,3 |
| | 320 | 83,4 | 96,5 | | | |
| | 340 | 88,7 | 96,1 | | | |
| | 350 | 91,1 | 95,7 | | | |
| **Beispiel 6** | 300 | 88,2 | 97,5 | II (extr) | 25,81 | 22,49 |
| | 320 | 92,7 | 97,6 | | | |
| | 350 | 98,4 | 96,7 | | | |
| | 360 | 99,2 | 96,4 | | | |
| **Beispiel 7** | 300 | 86,6 | 98,3 | II (extr) | 25,81 | 22,49 |
| | 320 | 91,8 | 98,2 | | | |
| | 350 | 97,7 | 97,4 | | | |
| | 360 | 98,6 | 97,1 | | | |

| | | | | | | |
|---|---|---|---|---|---|---|
| *: I: Spheralite 501A, Partikelgröße 2 ― 5 mm; II: Spheralite 509A, Partikelgröße 7 ― 15 µm; imp: imprägniert; press 4: in Tablettenform gepresst bei einem Druck von 4 t; mill: gemahlen (Partikelgröße nach dem Mahlen ≤ 250 µm); extr: extrudiert | | | | | | |

Weitere Versuchsbedingungen sind der folgenden Aufstellung zu entnehmen:
GHSV: 1300 h⁻¹ (bezogen auf Normbedingungen bei 0 °C und 1,013 bar gemäß DIN 1343)
LHSV: 0,4 h⁻¹ (bezogen auf flüssiges Methanol)
Massenverhältnis H₂S/MeOH: 1,9
Druck: 9 bar

Das erhaltene Reaktionsgemisch, umfassend die Produkte Methylmercaptan, Dimethylsulfid, Dimethyldisulfid und Dimethylether sowie die nicht umgesetzten Edukte Methanol und Schwefelwasserstoff wurde online gaschromatografisch analysiert.

Die Messergebnisse sind in Tabelle 1 sowie den Abbildungen 1 bis 3 dargestellt.

Es ist zu erkennen, dass ein durch das erfindungsgemäße Verfahren hergestellter Katalysator mit einem feinteiligen Trägermaterial gegenüber einem Katalysator, welcher durch Imprägnierung von Partikeln des Trägermaterials einer Größe von 2 mm und mehr hergestellt ist, bei gleicher Beladung einen höheren Umsatz bei einer gegebenen Temperatur aufweist (Vergleichsbeispiel 2 gegenüber dem erfindungsgemäßen Beispiel 4) und somit bei gleichem Umsatz eine höhere Selektivität für Methylmercaptan aufweist.

Weiterhin können nach dem erfindungsgemäßen Herstellungsverfahren Katalysatoren in Form von Extrudaten und Presslingen hergestellt werden, die eine hohe Beladung (beispielsweise über 45 Gew.-%) der oxidischen Zusammensetzung aufweisen, wodurch der Umsatz und die Selektivität der katalysierten Reaktionen weiter gesteigert werden kann. Dies ist bei Beladung über 45 Gew.-% für einen nach dem Imprägnierverfahren hergestellten Katalysator nicht möglich (Vergleichsbeispiel 1 und erfindungsgemäßes Beispiel 3 im Vergleich zu Vergleichsbeispiel 2 und dem erfindungsgemäßen Beispiel 4).

Durch Zugabe von anorganischen und/oder organischen Bindern, bevorzugt anorganischen Bindern, kann nicht nur die mechanische Festigkeit des erfindungsgemäßen Katalysators erhöht werden, sondern überraschenderweise auch eine weitere Steigerung der Selektivität und des Umsatzes bei einer bestimmten Temperatur beobachtet werden (Beispiel 6).

Es zeigt sich ferner, dass auch die Reihenfolge der Zugabe des Alkalimetallhydroxids und der oxidischen Wolframverbindung den Umsatz und die Selektivität der Reaktion bei einer bestimmten Temperatur beeinflussen (erfindungsgemäße Beispiele 6 und 7).

### 1.2. Beispiele 9 bis 13: Variation der Beladung

Beispiel 5 wurde jeweils mit erhöhter Beladung des Aluminiumoxids mit Wolfram(VI)oxid und Cäsiumhydroxid wiederholt. Die jeweilige Beladung des Katalysators sowie der hiermit bei einer bestimmten Temperatur erzielte Umsatz und die erreichte Selektivität in einem Anwendungsbeispiel gemäß Beispiel 8 sind in Tabelle 2 sowie der Abbildung 4 dargestellt.

**Tabelle 2**

| **Katalysator** | **T** | **Umsatz** | **Selektivität** | **Träger*** | **Beladung** | |
|---|---|---|---|---|---|---|
| | | | | | WO₃ | Cs₂O |
| | [°C] | [%] | [%] | | [Gew.-%] | [Gew.-%] |
| **Beispiel 9** | 300 | 81 | 97,23 | I (mill, press 4) | 20,5 | 19,9 |
| | 320 | 86,05 | 97,05 | | | |
| | 340 | 91,17 | 96,63 | | | |
| | 350 | 93,3 | 96,35 | | | |
| **Beispiel 10** | 300 | 83,05 | 97,2 | **I (mill, press 4)** | 21,8 | 21,2 |
| | 320 | 89,13 | 97 | | | |
| | 340 | 94 | 96,65 | | | |
| | 350 | 96,05 | 96,3 | | | |
| **Beispiel 11** | 300 | 83,3 | 97,7 | I (mill, press 4) | 23,1 | 22,5 |
| | 320 | 88,47 | 97,43 | | | |
| | 340 | 92,9 | 97,1 | | | |
| | 350 | 94,57 | 96,9 | | | |
| **Beispiel 12** | 300 | 90,15 | 97,25 | I (mill, press 4) | 24,5 | 23,8 |
| | 320 | 94,93 | 97,1 | | | |
| | 340 | 98,03 | 96,43 | | | |
| | 350 | 98,8 | 96,1 | | | |
| **Beispiel 13** | 300 | 83,3 | 97,4 | I (mill, press 4) | 25,8 | 25,1 |
| | 320 | 88,8 | 96,95 | | | |
| | 340 | 93,55 | 96,6 | | | |
| | 350 | 95,15 | 96,4 | | | |

| | | | | | | |
|---|---|---|---|---|---|---|
| *: I: Spheralite 501A, Partikelgröße 2 ― 5 mm; press 4: in Tablettenform gepresst bei 4 bar Druck; mill: gemahlen (Partikelgröße nach dem Mahlen ≤ 250 µm) | | | | | | |

Es ist zu erkennen, dass eine Erhöhung der Beladung einen positiven Einfluss auf den Umsatz und die Selektivität der katalysierten Reaktion bei einer gegebenen Temperatur hat und durch Erhöhung der Beladung sowohl Umsatz und Selektivität bei einer gegebenen Temperatur als auch die Selektivität der Umsetzung von Methanol und Schwefelwasserstoff zu Methylmercaptan in Gegenwart des erfindungsgemäßen Katalysators erhöht werden können.

### 1.3. Beispiele 14 bis 17: Einfluss des partikulären Trägermaterials

In den Beispielen 14 bis 17 wurden bei einer Beladung von 23,1 Gew.-% WO₃ und 22,5 Gew.-% Cs₂O verschiedene Verfahren zur Herstellung des erfindungsgemäßen Katalysators miteinander verglichen.

### Beispiel 14:

Beispiel 11 wurde mit gleicher Beladung wiederholt, wobei der Druck in der Tablettenpresse 15 t statt 4 t betrug.

### Beispiel 15:

Beispiel 3 wurde mit einer Beladung von 23,1 Gew.-% WO₃ und 22,5 Gew.-% Cs₂O wiederholt.

### Beispiel 16:

Beispiel 11 wurde mit gleicher Beladung wiederholt, wobei der Aluminiumoxidträger nicht vor Vermengung mit der Wolframsäure gemahlen wurde, sondern zusammen mit der Wolframsäure in einer Kugelmühle (Firma Haldenwanger Berlin) mit einem Schwinherr Multifix Antrieb für 2 Stunden gemahlen wurde.

### Beispiel 17:

Beispiel 16 wurde wiederholt, wobei das Trägermaterial mit der Wolframsäure für 65 Stunden statt 2 Stunden in der Kugelmühle gemahlen wurde.

Der Umsatz und die Selektivität der Umsetzung von Methanol und Schwefelwasserstoff zu Methylmercaptan wurde für die erfindungsgemäßen Katalysatoren der Beispiele 14 bis 17 gemäß dem Anwendungsbeispiel 8 ermittelt. Die Ergebnisse sind in Tabelle 3 und Abbildung 5 dargestellt.

**Tabelle 3**

| **Katalysator** | **T** | **Umsatz** | **Selektivität** | **Träger*** | **Beladung** | |
|---|---|---|---|---|---|---|
| | | | | | WO₃ | Cs₂O |
| | [°C] | [%] | [%] | | [Gew.-%] | [Gew.-%] |
| **Beispiel 14** | 300 | 85,2 | 97,35 | I (mill, press 15) | 23,1 | 22,5 |
| | 320 | 91 | 97,1 | | | |
| | 340 | 95,47 | 96,73 | | | |
| | 350 | 97,03 | 96,43 | | | |
| | 360 | 98,25 | 96 | | | |
| | 370 | 99,1 | 95,43 | | | |
| **Beispiel 15** | 300 | 88,45 | 97,3 | II (press 4) | 23,1 | 22,5 |
| | 320 | 93,53 | 97,33 | | | |
| | 340 | 97 | 96,85 | | | |
| | 350 | 98,13 | 96,33 | | | |
| **Beispiel 16** | 300 | 88,1 | 97,65 | I (ball mill 2, press 4) | 23,1 | 22,5 |
| | 320 | 92,75 | 97,6 | | | |
| | 340 | 96,7 | 97,25 | | | |
| | 350 | 98,05 | 96,75 | | | |
| **Beispiel 17** | 300 | 87,23 | 97,93 | I (ball mill 65, press 4) | 23,1 | 22,5 |
| | 320 | 91,7 | 97,7 | | | |
| | 340 | 96,05 | 97,35 | | | |
| | 350 | 97,3 | 96,95 | | | |
| | 360 | 98,5 | 96,65 | | | |

| | | | | | | |
|---|---|---|---|---|---|---|
| *: I: Spheralite 501A, Partikelgröße 2 ― 5 mm; II: Spheralite 509A, Partikelgröße 7 ― 15 µm; imp: imprägniert; press 4/15: in Tablettenform gepresst bei 4 bzw. 15 t Druck; ball mill 2/65: Trägermaterial mit Wolframsäure für 2 bzw. 65 h in einer Kugelmühle gemahlen; mill: gemahlen (Partikelgröße nach dem Mahlen ≤ 250 µm) | | | | | | |

Wie bereits die vorhergehenden Beispiele zeigen auch die Beispiele 14 bis 17, dass es mit der vorliegenden Erfindung möglich ist, Katalysatoren bereitzustellen, mit welchen für die Reaktion von Methanol und Schwefelwasserstoff zu Methylmercaptan auch bei einem Umsatz von mehr als 95 % eine Selektivität von über 95 % erhalten werden kann. So kann beispielsweise mit dem Katalysator gemäß Beispiel 14 bei einer Temperatur von 370 °C sogar bei einem Umsatz von über 99 % noch eine Selektivität von über 95 % erzielt werden.

Eine geringe Partikelgröße des Trägermaterials sowie eine intensive Durchmischung des Trägermaterials, der oxidischen Wolframverbindung und des Alkalihydroxids, beispielsweise in einer Kugelmühle gemäß Beispiel 16 oder 17, wirken sich bei ansonsten unveränderten Katalysatorparametern positiv auf den Umsatz und die Selektivität des Katalysators bei einer bestimmten Temperatur aus (Beispiele 16 und 17 im Vergleich zu Beispiel 11).

### 1.4. Beispiel 18: Energiedispersive Röntgenspektroskopie

Die Konzentrationsverteilung der Elemente Aluminium, Cäsium und Wolfram an der Oberfläche der Katalysatoren gemäß den Beispielen 1, 2 und 6 wurde mithilfe energiedispersiver Röntgenspektroskopie (EDX-Mapping) gemäß ISO 22309 (2006) sichtbar gemacht (Abbildungen 6 bis 8). Hierzu wurde ein Jeol 7600F Scanning Electron Microscope (SEM) (Jeol Ltd., Tokio, Japan) zusammen mit einem Oxford INCA Energy 400 Energy Dispersive X-Ray Analysis (EDX) System (Oxford Instruments, Abingdon, Großbritannien) verwendet. Die SEM Aufnahmen und die EDX-Mappings der ausgesuchten Elemente wurden bei 20 keV Primärelektronenstrahlenergie aufgenommen. Die geschnitteten, eingebetteten und polierten Katalysatorteilchen wurden mit einem 20 nm dünnen Kohlenstoffcoating für die elektronische Leitfähigkeit des Analysenelektronenstrahl bedeckt.

Überraschenderweise zeigte sich hierbei, dass die Elemente Cäsium und Wolfram in dem erfindungsgemäßen Verfahren, bei welchem das Trägermaterial bevorzugt zunächst mit Verbindungen des einen Elements vermischt wird, bevor die Verbindung des anderen Elements zu dieser Mischung hinzugegeben wird, homogener in dem Katalysator verteilt sind, als wenn Katalysatorpartikel nach dem aus dem Stand der Technik bekannten Imprägnierverfahren mit einer Lösung behandelt werden, welche eine Lösung beider Elemente in Form von Cäsiumwolframat enthält.

Als jeweilige Abbildung a) ist eine Materialkontrastabbildung eines Katalysatorteilchens gezeigt, in welcher bereits die weitgehende Homogenität (Abb. 8a: Beispiel 6) bzw. Inhomogenität (Abb. 6a) der Katalysatorstruktur bzw. der Verteilung der Elemente in demselben erkennbar wird. Die jeweiligen Abbildungen b) ― d) zeigen die Konzentration der Elemente Cäsium (Abb. b), Wolfram (Abb. c) und Aluminium (Abb. d) in dem Katalysatorpartikel.

Je heller eine Stelle in diesen Abbildungen erscheint, desto dichter ist das Material und damit desto höher die Konzentration des betreffenden Elements.

Es ist klar zu erkennen, dass der erfindungsgemäße Katalysator (Abb. 8 a bis d) eine deutlich homogene Verteilung der Elemente über den gesamten Katalysator, d. h. auch an der Oberfläche desselben, aufweist.

Um dies zu quantifizieren, wurde die Elementverteilung anhand einer Geraden im Querschnitt eines jeden Katalysatorteilchens mithilfe quantitativer EDX-Messungen in etwa 40 Quadratausschnitten mit jeweils 100 µm Seitenlänge bestimmt. Die Mittelpunkte der Quadrate liegen hierbei auf der Geraden und sind jeweils 100 µm vom Mittelpunkt des jeweils angrenzenden Quadrats entfernt. Der erste und der letzte Quadratmittelpunkt dieser Geraden liegen jeweils in etwa 100 µm vom Rand des Katalysatorkörpers entfernt. Die Verteilung dieser Messquadrate über den Katalysatorquerschnitt ist in den jeweiligen Abbildungen e) verdeutlicht.

Aus den Intensitäten der Signale in dem jeweiligen Quadraten wurde der jeweilige Anteil eines Elements an der Summe der Anteile aller Elemente in diesem Quadranten bestimmt (im folgenden als Konzentration bezeichnet), wobei die Summe in jedem Messpunkt jeweils 100% betrug. Aus den in den jeweiligen Quadraten bestimmten Konzentrationen an Cäsium und Wolfram wurde der Mittelwert über den Querschnitt der Probe, d. h. über die etwa 40 Einzelwerte gebildet. Der Quotient aus der experimentell bestimmten Konzentration an Cäsium oder Wolfram im jeweiligen Quadrat und dem Mittelwert dieser Konzentration über den Querschnitt der Probe, multipliziert mit dem Faktor 100 wird als normierte Konzentration Cₙₒᵣₘ bezeichnet, aus der mithilfe der Tabellenkalkulation Excel (Microsoft Office Excel 2003, Microsoft Corporation, Redmont, U.S.A.) die Standardabweichung Stabw, die mittlere Abweichung Mittelabw, der jeweils größte und kleinste gemessene Wert (Max bzw. Min) sowie die Differenz aus Maximum und Minimum der normierten Konzentrationen Δ (Max-Min) über den Querschnitt der jeweiligen Probe berechnet wurden. Die Ergebnisse sind in den Tabellen 4 (nicht erfindungsgemäßes Beispiel 1), 5 (nicht erfindungsgemäßes Beispiel 2) und 6 (erfindungsgemäßes Beispiel 6) dargestellt.

**Tabelle 4: Quantifizierung der normierten Konzentration an Cäsium und Wolfram über den Katalysatorquerschnitt in Beispiel 1**

| **MP** | **Cₙₒᵣₘ (Cs) [%]** | **Cₙₒᵣₘ (W) [%]** | **Cₙₒᵣₘ (Cs) / Cₙₒᵣₘ(W)** |
|---|---|---|---|
| **1** | 103,31 | 159,65 | **0,65** |
| 2 | 85,44 | 122,15 | 0,70 |
| 3 | 80,13 | 112,44 | 0,71 |
| 4 | 76,01 | 105,57 | 0,72 |
| 5 | 87,64 | 113,18 | 0,77 |
| 6 | 89,13 | 113,61 | 0,78 |
| 7 | 68,99 | 90,78 | 0,76 |
| 8 | 94,38 | 113,92 | 0,83 |
| 9 | 71,26 | 98,21 | 0,73 |
| 10 | 122,95 | 103,53 | 1,19 |
| 11 | 116,79 | 100,56 | 1,16 |
| 12 | 113,24 | 108,29 | 1,05 |
| 13 | 117,99 | 98,21 | 1,20 |
| 14 | 111,89 | 96,66 | 1,16 |
| 15 | 105,44 | 100,74 | 1,05 |
| 16 | 90,41 | 72,83 | 1,24 |
| 17 | 94,87 | 77,48 | 1,22 |
| 18 | 92,11 | 70,17 | 1,31 |
| 19 | 105,58 | 75,19 | 1,40 |
| 20 | 97,92 | 69,86 | 1,40 |
| 21 | 101,47 | 74,01 | 1,37 |
| 22 | 94,52 | 68,75 | 1,37 |
| 23 | 90,97 | 72,40 | 1,26 |
| 24 | 96,08 | 67,88 | 1,42 |
| **25** | 107,85 | 84,96 | 1,27 |
| 26 | 112,11 | 92,57 | 1,21 |
| 27 | 106,79 | 94,74 | 1,13 |
| 28 | 116,15 | 99,32 | 1,17 |
| 29 | 120,05 | 99,20 | 1,21 |
| 30 | 120,54 | 103,71 | 1,16 |
| 31 | 121,82 | 109,16 | 1,12 |
| 32 | 127,70 | 107,61 | 1,19 |
| 33 | 111,54 | 116,46 | 0,96 |
| 34 | 63,68 | 99,94 | 0,64 |
| 35 | 100,19 | 116,71 | 0,86 |
| 36 | 97,29 | 116,09 | 0,84 |
| 37 | 82,75 | 106,81 | 0,77 |
| 38 | 90,62 | 119,74 | 0,76 |
| 39 | 112,39 | 146,91 | 0,77 |
| Stabw | 15,97 | 20,51 | 0,25 |
| **Mittelabw** | 13,12 | 15,22 | 0,22 |
| **Max** | 127,70 | 159,65 | 1,42 |
| **Min** | 63,68 | 67,88 | 0,64 |
| **Δ (Max-Min)** | 64,03 | 91,77 | 0,78 |

**Tabelle 5: Quantifizierung der normierten Konzentration an Cäsium und Wolfram über den Katalysatorquerschnitt in Beispiel 2**

| **MP** | **Cₙₒᵣₘ (Cs) [%]** | **Cₙₒᵣₘ (W) [%]** | **Cₙₒᵣₘ (Cs) / Cₙₒᵣₘ(W)** |
|---|---|---|---|
| 1 | 166,40 | 213,91 | 0,78 |
| 2 | 147,14 | 187,15 | 0,79 |
| 3 | 128,88 | 153,28 | 0,84 |
| 4 | 91,08 | 101,77 | 0,90 |
| 5 | 99,89 | 94,92 | 1,05 |
| 6 | 98,05 | 97,83 | 1,00 |
| 7 | 98,05 | 91,19 | 1,08 |
| 8 | 97,76 | 93,27 | 1,05 |
| 9 | 91,65 | 89,32 | 1,03 |
| 10 | 94,85 | 88,36 | 1,07 |
| 11 | 95,70 | 82,69 | 1,16 |
| 12 | 93,29 | 84,35 | 1,11 |
| 13 | 91,87 | 88,49 | 1,04 |
| 14 | 96,41 | 81,24 | 1,19 |
| **15** | 95,13 | 85,25 | 1,12 |
| 16 | 97,83 | 85,11 | 1,15 |
| 17 | 95,56 | 85,25 | 1,12 |
| 18 | 91,51 | 85,59 | 1,07 |
| 19 | 97,48 | 78,33 | 1,24 |
| 20 | 92,58 | 82,20 | 1,13 |
| 21 | 92,01 | 88,91 | 1,03 |
| 22 | 92,43 | 83,52 | 1,11 |
| 23 | 89,38 | 82,48 | 1,08 |
| 24 | 87,11 | 86,77 | 1,00 |
| 25 | 91,44 | 82,69 | 1,11 |
| 26 | 93,50 | 79,51 | 1,18 |
| 27 | 92,36 | 80,96 | 1,14 |
| 28 | 93,93 | 85,73 | 1,10 |
| 29 | 94,49 | 88,01 | 1,07 |
| 30 | 98,90 | 88,56 | 1,12 |
| 31 | 98,26 | 90,43 | 1,09 |
| 32 | 95,35 | 86,35 | 1,10 |
| 33 | 95,63 | 91,88 | 1,04 |
| 34 | 93,00 | 91,88 | 1,01 |
| 35 | 91,79 | 95,34 | 0,96 |
| 36 | 95,28 | 97,07 | 0,98 |
| 37 | 95,63 | 102,74 | 0,93 |
| 38 | 86,32 | 103,77 | 0,83 |
| 39 | 95,13 | 106,12 | 0,90 |
| 40 | 141,32 | 170,14 | 0,83 |
| 41 | 125,61 | 167,66 | 0,75 |
| **Stabw** | 16,88 | 31,20 | 0,12 |
| **Mittelabw** | 10,21 | 19,83 | 0,09 |
| **Max** | 166,40 | 213,91 | 1,24 |
| **Min** | 86,32 | 78,33 | 0,75 |
| **Δ (Max-Min)** | 80,07 | 135,58 | 0,50 |

**Tabelle 6: Quantifizierung der normierten Konzentration an Cäsium und Wolfram über den Katalysatorquerschnitt in Beispiel 6**

| **MP** | **Cₙₒᵣₘ (Cs) [%]** | **Cₙₒᵣₘ (W) [%]** | **Cₙₒᵣₘ (Cs) / Cₙₒᵣₘ (W)** |
|---|---|---|---|
| 1 | 112,60 | 107,38 | 1,05 |
| 2 | 110,31 | 99,52 | 1,11 |
| 3 | 102,72 | 97,71 | 1,05 |
| 4 | 103,75 | 97,24 | 1,07 |
| 5 | 112,52 | 97,71 | 1,15 |
| 6 | 106,75 | 108,11 | 0,99 |
| 7 | 109,67 | 110,64 | 0,99 |
| 8 | 95,85 | 99,31 | 0,97 |
| 9 | 102,48 | 112,97 | 0,91 |
| 10 | 103,19 | 105,73 | 0,98 |
| 11 | 97,82 | 101,85 | 0,96 |
| 12 | 105,72 | 99,26 | 1,07 |
| 13 | 100,43 | 99,57 | 1,01 |
| 14 | 99,01 | 99,78 | 0,99 |
| 15 | 99,80 | 101,49 | 0,98 |
| 16 | 107,70 | 101,74 | 1,06 |
| 17 | 96,56 | 92,59 | 1,04 |
| 18 | 105,56 | 105,16 | 1,00 |
| 19 | 108,73 | 108,42 | 1,00 |
| 20 | 102,56 | 87,05 | 1,18 |
| 21 | 99,56 | 100,14 | 0,99 |
| 22 | 92,37 | 91,35 | 1,01 |
| 23 | 104,06 | 109,45 | 0,95 |
| 24 | 111,10 | 106,61 | 1,04 |
| 25 | 91,18 | 94,55 | 0,96 |
| 26 | 93,24 | 90,73 | 1,03 |
| 27 | 85,34 | 86,85 | 0,98 |
| 28 | 73,88 | 89,28 | 0,83 |
| 29 | 77,04 | 91,35 | 0,84 |
| 30 | 100,35 | 95,23 | 1,05 |
| 31 | 103,98 | 104,80 | 0,99 |
| 32 | 88,50 | 97,81 | 0,90 |
| 33 | 92,61 | 92,33 | 1,00 |
| 34 | 96,32 | 102,68 | 0,94 |
| 35 | 84,86 | 85,40 | 0,99 |
| 36 | 87,79 | 92,85 | 0,95 |
| 37 | 104,77 | 102,78 | 1,02 |
| 38 | 106,91 | 107,38 | 1,00 |
| 39 | 109,99 | 108,57 | 1,01 |
| 40 | 112,44 | 116,64 | 0,96 |
| **Stabw** | 9,48 | 7,62 | 0,07 |
| **Mittelabw** | 7,41 | 6,13 | 0,05 |
| **Max** | 112,60 | 116,64 | 1,18 |
| **Min** | 73,88 | 85,40 | 0,83 |
| **Δ (Max-Min)** | 38,72 | 31,24 | 0,35 |

Es ist eindeutig zu erkennen, dass in dem erfindungsgemäßen Katalysator gemäß Beispiel 6 Standardabweichung und Mittelabweichung sowohl der Cäsiumverteilung als auch der Wolframverteilung über den Katalysator deutlich geringer sind. Auch die Differenz zwischen dem Maximum und dem Minimum der jeweils ermittelten Konzentration ist in dem erfindungsgemäßen Katalysator deutlich geringer. Darüber hinaus ist auch das Verhältnis der normierten Cäsiumkonzentration zur normierten Wolframkonzentration Cₙₒᵣₘ (Cs) / Cₙₒᵣₘ (W) in dem erfindungsgemäßen Katalysator über den Katalysatorquerschnitt deutlich gleichmäßiger, wie sich aus der Standard- und der Mittelabweichung dieses Wertes sowie der Differenz aus Maximal- und Minimalwert für die jeweiligen Katalysatoren ergibt.

Diese quantitativen Messungen bestätigen die deutlich homogenere Verteilung der Elemente Cäsium und Wolfram über den gesamten Katalysator.

## Patentansprüche

1. Verfahren zur Herstellung eines Katalysators, umfassend ein Trägermaterial und eine oxidische Zusammensetzung, welche neben Sauerstoff mindestens ein Alkalimetall und Wolfram enthält, besagtes Verfahren umfassend die Schritte:
1) Bereitstellen mindestens eines Trägermaterials, einer oxidischen Wolframverbindung und mindestens einer separaten Alkalimetallverbindung,
2) Vermischen des Trägermaterials mit der oxidischen Wolframverbindung und der mindestens einen separaten Alkalimetallverbindung, um eine Katalysatormasse zu erhalten und
3) Formen der erhaltenen Katalysatormasse.

2. Verfahren gemäß Anspruch 1, wobei die oxidische Wolframverbindung ausgewählt ist aus der Gruppe, bestehend aus Wolframtrioxid (WO₃), Wolframsäure (WO₃ • H₂O), Metawolframsäure, Parawolframsäure, Isopolywolframsäuren, Heteropolywolframsäuren, Ammoniumsalzen derselben, Hydraten derselben oder Mischungen derselben und bevorzugt Wolframsäure, Ammoniumorthowolframat, Ammoniummetawolframat oder Ammoniumparawolframat darstellt.

3. Verfahren gemäß Anspruch 1 oder 2, wobei die mindestens eine Alkalimetallverbindung eine basische Alkalimetallverbindung ist, bevorzugt ausgewählt aus der Gruppe, bestehend aus Hydroxiden und Carbonaten von Alkalimetallen, besonders bevorzugt aus der Gruppe, bestehend aus Hydroxiden und Carbonaten von Natrium, Kalium, Cäsium und Rubidium.

4. Verfahren gemäß einem der Ansprüche 1 bis 3, wobei das Trägermaterial ein oxidisches anorganisches Trägermaterial ist, bevorzugt ausgewählt aus der Gruppe enthaltend Aluminiumoxid, Siliciumdioxid, Titandioxid, Zirconoxid, amorphe Aluminosilikate und Gemische derselben und bevorzugt in Form von Partikeln mit einer Größe von weniger als 1.000 µm eingesetzt wird.

5. Verfahren gemäß einem der Ansprüche 1 bis 4, wobei die oxidische Wolframverbindung und die separate Alkalimetallverbindung nacheinander zu dem Trägermaterial hinzugegeben werden und die oxidische Wolframverbindung bevorzugt als Feststoff zu dem Trägermaterial bzw. dem Gemisch aus Trägermaterial und der mindestens einen Alkalimetallverbindung gegeben wird.

6. Verfahren gemäß einem der Ansprüche 1 bis 5, wobei in einem oder beiden der Schritte 2 und 3 mindestens ein organischer und/oder anorganischer Binder hinzugesetzt wird.

7. Verfahren gemäß einem der Ansprüche 1 bis 6, wobei das Formen des Katalysators durch Extrusion oder Pressen erfolgt und das Verfahren bevorzugt die folgenden zusätzlichen Schritte umfasst:
(i) Trocknen der Katalysatormasse und/oder des geformten Katalysators
(ii) Calcinieren der Katalysatormasse und/oder des geformten Katalysators.

8. Katalysator, welcher ein Trägermaterial und eine oxidische Zusammensetzung, enthaltend neben Sauerstoff mindestens ein Alkalimetall und Wolfram, umfasst, hergestellt aus Partikeln des Trägermaterials mit einer Partikelgröße von weniger als 1.000 µm.

9. Katalysator gemäß Anspruch 8, in welchem die Standardabweichung Stabw der normierten Wolframkonzentration (Cₙₒᵣₘ (W)) über den Querschnitt des Katalysators weniger als 20 beträgt, ermittelt durch quantitative EDX-Messung gemäß ISO 22309 (2006) in mindestens zehn quadratischen Messpunkten mit jeweils 100 µm Seitenlänge, deren Mittelpunkte auf einer Gerade liegen und jeweils 100 µm vom jeweils angrenzenden Quadratmittelpunkt entfernt sind, wobei der jeweils erste und letzte Quadratmittelpunkt jeweils 100 µm vom Rand des Katalysatorquerschnitts entfernt ist.

10. Katalysator gemäß Anspruch 8 oder 9, **dadurch gekennzeichnet, dass** die oxidische Zusammensetzung eine Zusammensetzung der allgemeinen Formel AₓWO_{y} darstellt, in welcher bedeuten
A: mindestens ein Alkalimetall, bevorzugt ausgewählt aus der Gruppe, bestehend aus Natrium, Kalium, Rubidium und Cäsium,
x: molarer Anteil des mindestens einen Alkalimetalls im Verhältnis zu Wolfram in der Zusammensetzung, welcher bevorzugt im Bereich von 2:1 zu 0,8:1 liegt und
y: molarer Anteil Sauerstoff in der Zusammensetzung, welcher bevorzugt im Bereich von 3,4 bis 4 liegt.

11. Katalysator gemäß irgendeinem der Ansprüche 8 bis 10, hergestellt durch ein Verfahren, welches einen Schritt des Vermengens einer festen Wolframverbindung, bevorzugt einer festen oxidischen Wolframverbindung, mit dem Trägermaterial umfasst.

12. Katalysator gemäß irgendeinem der Ansprüche 8 bis 11, wobei der Anteil der oxidischen Zusammensetzung aus Alkalimetall und Wolfram in dem Katalysator mehr als 15 Gew.-%, bevorzugt mehr als 25 Gew.-%, weiterhin bevorzugt mehr als 36 Gew.-%, besonders bevorzugt mehr als 40 Gew.-% und insbesondere mehr als 45 Gew.-% beträgt, bezogen auf das Gesamtgewicht des Katalysators.

13. Katalysator gemäß irgendeinem der Ansprüche 8 bis 12, wobei das Trägermaterial mindestens eine oxidische anorganische Verbindung umfasst, bevorzugt ausgewählt aus der Gruppe enthaltend Aluminiumoxid, Siliciumdioxid, Titandioxid, Zirkonoxid, amorphe Aluminosilikate und Gemische derselben.

14. Katalysator gemäß irgendeinem der Ansprüche 8 bis 13, ferner umfassend mindestens ein organisches und/oder anorganisches Bindemittel.

15. Katalysator gemäß irgendeinem der Ansprüche 8 bis 14, hergestellt nach einem Verfahren gemäß irgendeinem der Ansprüche 1 bis 7.

16. Verfahren zur Herstellung von Alkylmercaptanen durch Umsetzung von Alkanolen mit Schwefelwasserstoff in Gegenwart eines Katalysators gemäß irgendeinem der Ansprüche 8 bis 15 oder in Gegenwart eines Katalysators hergestellt durch ein Verfahren gemäß irgendeinem der Ansprüche 1 bis 7
